# EUROPÄISCHE PATENTSCHRIFT

(11) **EP 3 957 280 B1**
(45) Veröffentlichungstag und Bekanntmachung des Hinweises auf die Patenterteilung: **16.11.2022**
(21) Anmeldenummer: 20191372.0
(22) Anmeldetag: 17.08.2020
(51) Int. Cl.: A61F 2/30, A61F 2/36, A61F 2/38, A61F 2/40, A61F 2/46, A61P 31/10, B29C 33/00, B29C 33/30, B29C 39/26, A61B 17/56, B29C 33/12, B29K 33/00, B29L 31/00

(54) **VORRICHTUNG UND VERFAHREN ZUR HERSTELLUNG VON SPACERN**
DEVICE AND METHOD FOR PRODUCING SPACERS
DISPOSITIF ET PROCÉDÉ DE FABRICATION D'ESPACEURS

(43) Veröffentlichungstag der Anmeldung: 23.02.2022
(73) Patentinhaber: Heraeus Medical GmbH, 61273 Wehrheim (DE)
(72) Erfinder: Vogt, Sebastian Dr., 61273 Wehrheim (DE); Kluge, Thomas Dr., 61273 Wehrheim (DE)
(74) Vertreter: Schultheiss & Sterzel Patentanwälte PartG mbB

(56) Entgegenhaltungen:
- EP-A1- 2 522 310
- DE-B4-102015 104 704
- US-A1- 2009 157 189
- US-A1- 2019 290 833
- US-B1- 6 361 731

## Beschreibung

Die Erfindung ist definiert durch die Ansprüche 1, 11 und 13 und betrifft eine Vorrichtung , ein Set und ein Verfahren zum Herstellen eines Spacers durch Aushärten von Knochenzementteig. Der Spacer ist als temporärer Platzhalter dazu vorgesehen, im medizinischen Anwendungsbereich ein Gelenk oder einen Teil eines Gelenks umfassend eine artikulierende Oberfläche eines Gelenkkopfs temporär zu ersetzen. Bevorzugt ist der Spacer zum temporären Ersetzen eines Hüftgelenks oder eines Schultergelenks geeignet und vorgesehen, besonders bevorzugt zum temporären Ersetzen eines Hüftgelenks. Dementsprechend ist die Vorrichtung bevorzugt zum Herstellen eines Hüftgelenkspacers oder eines Schultergelenkspacers vorgesehen. Die Erfindung betrifft auch ein Set und ein Verfahren zur Herstellung eines solchen Spacers mit einer solchen Vorrichtung.

Gegenstand der Erfindung ist somit insbesondere eine Vorrichtung in Form einer zweiteiligen Gießform zur Herstellung von einteiligen Hüft- und Schulterspacern, wobei die Gießform eine Kopfform und eine Schaftform aufweist und ein Metallkern zum Aufbau des Spacers verwendet wird. Hüft- und Schulterspacer sind als temporäre Platzhalter (Spacer) im Rahmen von zweizeitigen Revisionen von infizierten Hüft- und Schultertotalgelenkendoprothesen für die Interimsphase bestimmt. Die Vorrichtung ist für die Herstellung von Hüft- und Schulterspacern mit niedrigviskosem und hochviskosem Polymethylmethacrylat-Knochenzementteig geeignet. Gelenk-Endoprothesen, wie Hüftgelenk-Endoprothesen und Schultergelenk-Endoprothesen, werden in großem Umfang weltweit implantiert. Leider kommt es in einem geringen Prozentsatz vor, dass Gelenk-Endoprothesen von mikrobiellen Keimen, besonders Grampositiven Bakterien als auch Gram-negativen Bakterien und in sehr geringem Umfang von Hefen und Pilzen, besiedelt werden. Diese mikrobiellen Keime, hauptsächlich typische Hautkeime wie *Staphylococcus aureus* und *Staphylococcus epidermidis*, können während einer chirurgischen Operation (OP) in den Patienten gelangen. Daneben ist es auch möglich, dass mikrobielle Keime hämatogen Gelenk-Endoprothesen erreichen. Bei einer Besiedlung von Gelenk-Endoprothesen mit mikrobiellen Keimen wird das umliegende Knochen- und Weichgewebe mit infiziert und von den mikrobiellen Keimen geschädigt.

Stand der Technik sind vor allem zwei Behandlungsverfahren für infizierte Gelenk-Endoprothesen, die einzeitige septische Revision und die zweizeitige septische Revision. Bei der einzeitigen Revision wird innerhalb einer OP zuerst die infizierte Gelenk-Endoprothesen entfernt, anschließend radikal debridiert und dann wird eine Revisions-Gelenk-Endoprothese implantiert.

Bei zweizeitigen septischen Revisionen wird in einer ersten OP zuerst die infizierte Gelenk-Endoprothese entfernt, dann wird debridiert und anschließend erfolgt die Implantation eines Spacers. Ein Hüftgelenkspacer besteht aus einem Schaft, einem Kragen, einem Hals und einem Kugelkopf und ist den Hüftgelenk-Endoprothesen in Form und Größe nachgebildet. Analog ist ein Schultergelenkspacer einer Schultergelenk-Endoprothesen in Form und Größe nachgebildet. Der Spacer wird mit Knochenzement an dem jeweiligen Knochen verankert, also beispielsweise bei Hüftgelenkspacern am proximalen Femur beziehungsweise im Femurkanal verankert. Der Spacer verbleibt bis zu mehreren Wochen im Patienten bis die Entzündung abgeklungen ist und die klinischen Entzündungsmarker zurückgegangen sind. Dann wird in einer zweiten OP der Spacer entfernt und nach erneutem Debridement eine Revisions-Gelenk-Endoprothese implantiert.

Im Rahmen von zweizeitigen septischen Wechseloperationen von Hüft- und Schultertotalgelenkendoprothesen sind Spacer als temporäre Platzhalter in der Interimsphase von wesentlicher Bedeutung. Bei der intraoperativen Herstellung dieser Spacer kann vom medizinischen Personal je nach vorliegenden Antibiogramm der für die Infektionen ursächlichen mikrobiellen Keime ein oder mehrere speziell auf die Keime abgestimmtes Antibiotikum oder mehrere Antibiotika dem Polymethylmethacrylat-Knochenzement zugesetzt werden.

Bei Spacern werden dem Zementpulver vor der eigentlichen Spacerherstellung Antibiotika zugesetzt. Mit diesem antibiotisch modifizierten Knochenzementpulver wird anschließend durch Beimischen von Monomerflüssigkeit ein Knochenzementteig hergestellt und aus diesem Knochenzementteig werden Spacer gegossen, die dann durch Polymerisation mit Hilfe der dem Zementpulver zugesetzten Monomerflüssigkeit aushärten. Der Knochenzementteig schließt dabei die Antibiotika im Wesentlichen ein. Die in den oberflächennahen Bereichen befindlichen Antibiotika-Partikel werden bei Einwirkung von Körperflüssigkeiten, wie Wundsekret, herausgelöst. Die Wirkstofffreisetzung ist initial am höchsten und nimmt dann im Verlauf von mehreren Tagen ab.

Die US 2010/0042213 A1 offenbart eine Hüftgelenkprothese mit einem Reservoir einer Flüssigkeit im Inneren des Implantats. Aus der WO 2017/178951 A1 ist ein Hüftspacer mit Vertiefungen bekannt, wobei in den Vertiefungen eine Substanz zur Behandlung des Knochens eingebracht werden kann. In dem Patent US 6 245 111 B1 wird eine Hüftgelenksprothese vorgeschlagen, deren Oberflächen mit einem Antibiotikum beschichtet sind. Das Patent US 5 681 289 offenbart eine Einrichtung zum Verteilen eines flüssigen Wirkstoffs mit Hilfe einer Blase im Inneren der Einrichtung. Keine der genannten Prothesen ist zum Erzeugen eines Spülkreislaufs geeignet. In der EP 1 991 170 B1 und der US 2011/0015754 A1 werden ein Hüftgelenkspacer beschrieben, die Wirkstoffe enthalten. Die US 2019/0290833 A1 offenbart einen spülbaren Hüftgelenkspacer, mit dem ein Flüssigkeitskreislauf erzeugbar ist. In der WO 2016/205077 A1 und der US 8 900 322 B2 werden weitere Spacer mit einer Spülfunktion beschrieben.

Es ist bekannt, mit Antibiotika ausgerüstete Spacer zu verwenden. Spacer können einerseits vom OP-Personal während der OP selbst aus PMMA-Knochenzementpulver, Antibiotika und Monomerflüssigkeit hergestellt werden, zum Beispiel mit einer Spacerform, wie sie beispielsweise in den Patenten DE 10 2015 104 704 B4 oder EP 2 617 393 B1 beschrieben sind, und andererseits ist es auch üblich, industriell aus Knochenzement vorgefertigte Hüftgelenkspacer einzusetzen.

Für die intraoperative Herstellung von Spacern mit Polymethylmethacrylat-Knochenzement sind Kunststoffgießformen üblich. Kunststoffgießformen zur intraoperativen Herstellung von einteiligen Hüftspacern werden in der US 6 361 731 B1 beschrieben. Diese Gießformen sind transparent und haben zwei separate Einfüllöffnungen. Dadurch kann auch hochviskoser Knochenzementteig mit geringem Druck in die Gießform eingebracht werden, weil die Fließwege des Knochenzementteigs relativ kurz sind. Bei der Verwendung von nicht hochviskosem Knochenzementteig besteht die Gefahr, dass Knochenzementteig nach erfolgter Füllung der Gießform vor der einsetzenden Aushärtung wieder aus den Einfüllöffnungen herausfließt. Diese Gießformen werden mit unterschiedlichen Spacerkopfdurchmessern angeboten. Der Durchmesser des Spacerkopfs ist nicht variabel einstellbar. Der medizinische Anwender kann nur zwischen vorgegebenen Spacerkopfgrößen auswählen. Wünschenswert wäre es, wenn der medizinische Anwender möglichst mit einer Gießform für den Schaft zwischen verschiedenen Spacerkopfgrößen wählen könnte.

In einer Weiterentwicklung wurden in den Patentschriften US 7 789 646 B2, US 8 480 389 B2 und US 8 801 983 B2 mehrteilige Gießformen für die Herstellung modularer Hüftspacer vorgeschlagen. Diese modularen Hüftspacer bestehen aus einem Spacerkopf und einem separaten Schaft. Es stehen dazu Gießformen für den Spacerkopf mit unterschiedlichen Spacerkopfdurchmessern zur Verfügung. Das bedeutet, die Gießform des Schafts wird mit der Gießform des Spacerkopfs, welche den ausgewählten Durchmesser besitzt, verbunden. Die so zusammengesetzte Gießform ist dann einteilig und besitzt an der Einfüllöffnung ein Gewinde zur Verbindung der Gießform mit einer Zementkartusche. In einer anderen Variante gemäß der US 7 637 729 B2 werden eine Gießform für die Herstellung des Schafts und eine separate Gießform für den Spacerkopf verwendet. Nach erfolgter Aushärtung und Entformung werden die beiden Spacerkomponenten zusammengesteckt. Im Patent US 7 789 646 B2 wird eine modulare Gießform beschrieben, bei der mit einem Stöpsel die Einfüllöffnung der Gießform verschlossen werden kann, wenn der Zementteig in die Gießform eingebracht wurde. Zuvor muss jedoch die Gießform von der Zementkartusche abgeschraubt werden. Bei Verwendung von niedrigviskosem Zementteig ist es daher möglich, wenn die Gießform ungünstig gehalten wird, dass Zementteig während der Trennung der Gießform von der Zementkartusche ausläuft, bevor der Stöpsel eingeschraubt ist. Bei Verwendung von nicht hochviskosem Knochenzementteig ist es daher möglich, wenn die Gießform ungünstig gehalten wird, dass Knochenzementteig während der Trennung der Gießform von der Knochenzementkartusche ausläuft, bevor der Stöpsel eingeschraubt beziehungsweise eingesteckt ist. In jedem Fall können ungewollt Lufteinschlüsse in der Spacerform dadurch entstehen, dass sich der Knochenzementteig von der Innenwandung der Gießform löst.

Problematisch ist bei den aufgeführten Vorrichtungen, dass der Knochenzementteig eine relativ niedrige Viskosität aufweisen muss, damit der Metallkern vom Knochenzementteig umflossen und die Gießform vollständig vom Zementteig ausgefüllt werden kann.

In der US 2007/0222114 A1 wird eine Hüftspacerform beschrieben. Diese Spacerform besteht aus einer Vielzahl von Formsegmenten, die miteinander verbunden werden. Durch die Vielzahl der Segmente kann die Spacerform recht genau an die anatomischen Gegebenheiten des Patienten angepasst werden. Die Spacerform-Segmente werden mittels Schraubschellen aneinandergefügt. Durch Kanäle in der Spacerform wird ein PMMA-Knochenzementteig (Polymethylmethacrylat-Knochenzementteig) eingeführt. Durch den komplexen Aufbau der Gießform ist es sehr aufwändig, die Spacerform-Segmente zusammenzufügen und nach erfolgter Aushärtung des PMMA-Knochenzementteigs den Hüftspacer zu entnehmen.

In der WO 2009/073 781 A2 wird eine Spacerform für einen Hüftspacer vorgeschlagen, die aus zwei Teilen besteht, die in Bezug zueinander verschoben werden können, um die Länge des Schafts anpassen zu können. Eine weitere Gießform ist in der EP 2 522 310 A1 offenbart. Diese Vorrichtung besteht aus wenigstens zwei Teilen, wobei in einem ersten Teil eine Einschubabschnitt und im zweiten Teil eine Einschubaufnahme angeordnet sind. Beide Teile sind in einander steckbar und bilden eine Gießform für die Herstellung des Schafts des Hüftspacers. In der EP 2 787 928 A1 wird eine komplexe Gießform beschrieben. Diese ermöglicht die Herstellung von Hüftspacern mit unterschiedlichen Kugelköpfen. Die Fixierung der Elemente der Gießform erfolgt über Verbindungselemente.

Die Aufgabe der vorliegenden Erfindung besteht also darin, die Nachteile des Stands der Technik zu überwinden. Insbesondere besteht die Aufgabe der Erfindung in der Entwicklung einer einfachen und kostengünstigen Vorrichtung und eines Sets zum Herstellen eines einteiligen Spacers durch Aushärten von Knochenzementteig und in der Entwicklung eines einfach und kostengünstig durchführbaren Verfahrens zum Herstellen eines einteiligen Spacers durch Aushärten von Knochenzementteig, mit dem vom medizinischen Personal im OP unter Verwendung von Knochenzementteig, insbesondere von Polymethylmethacrylat-Knochenzementteig, einteilige Spacer, insbesondere Hüft- und Schulterspacer, hergestellt werden können. Hüft- und Schulterspacer sind ähnlich aufgebaut. Sie bestehen aus einem Schaft und einem Spacerkopf sowie einem Hals, der den Schaft und den Spacerkopf verbindet. Insbesondere soll eine Herstellung von Spacern mit unterschiedlichen, vorgegebenen Kopfdurchmessern und unterschiedlichen Längen des Halses möglich sein, wobei vom medizinischen Personal der Offset (die Länge des Halses), also der Abstand der Kopfs des Spacers zum Schaft des herzustellenden Spacers an der Gießform individuell einstellbar sein soll.

Es soll möglich sein, sowohl mit (Polymethylmethacrylat-)Knochenzementteig niedriger Viskosität als auch mit hoher Viskosität Spacer, insbesondere Hüftspacer herzustellen. Insbesondere soll es möglich sein, mit hochviskosem Zementteig ohne besonderen manuellen Krafteinsatz Spacer herzustellen. Die Gießform soll aus maximal zwei hohlen Kunststoffteilen aufgebaut sein, die möglichst kostengünstig, beispielsweise durch Kunststoffspritzgießen oder durch Tiefziehen von Kunststofffolien in einfacher Weise hergestellt werden können. Die Gießform soll einen Metallkern zur mechanischen Verstärkung des Hüftspacers aus einem biokompatiblen Metall enthalten. Die zu entwickelnde Gießform soll keinen Anguss oder mehrere Angüsse haben. Die herzustellenden Spacer sollen daher so beschaffen sein, dass sie keinen Anguss besitzen. Dadurch soll eine aufwendige mechanische Nachbearbeitung der hergestellten Spacer vermieden werden. Weiterhin soll ein Set beziehungsweise Kit entwickelt werden, mit dem es möglich ist, Spacer, insbesondere Hüftspacer, mit unterschiedlichen Kopfgrößen und Offsets entsprechend den jeweils individuellen anatomischen Verhältnissen der Patienten herzustellen. Es soll außerdem ein möglichst einfaches Verfahren zur Herstellung von Hüftspacern beschrieben werden.

Daher soll eine Vorrichtung, insbesondere mit einer Gießform und einem Metallkern, entwickelt werden, die grundsätzlich für die Herstellung von Hüft- und Schulterspacern bei möglichst einfachem und kostengünstigem Aufbau geeignet ist. Ein Metallkern soll zur mechanischen Stabilisierung im Inneren des Hüft- und Schulterspacer angeordnet sein beziehungsweise werden. Es soll möglich sein, sowohl mit (Polymethylmethacrylat-)Knochenzementteig niedriger beziehungsweise nicht hoher Viskosität aber auch mit hoher Viskosität Spacer herzustellen. Für die vollständige Füllung einer Gießform mit einem hochviskosem Knochenzementteig ist ein üblicherweise hoher Einpressdruck erforderlich. Es wäre hilfreich, wenn das vermieden werden könnte.

Die Aufgaben der Erfindung werden gelöst durch eine Vorrichtung zum Herstellen eines Spacers durch Aushärten von Knochenzementteig, wobei der Spacer dazu vorgesehen ist, im medizinischen Bereich ein Gelenk oder einen Teil eines Gelenks umfassend eine artikulierende Oberfläche eines Kopfs des Gelenks temporär zu ersetzen, insbesondere eines Hüftgelenks oder eines Schultergelenks temporär zu ersetzen, die Vorrichtung aufweisend eine Schaftform zum Formen eines Schafts des Spacers aus Knochenzementteig, wobei die Schaftform in ihrem Inneren einen Innenraum aufweist, wobei der Innenraum über eine proximale Öffnung an einer proximalen Seite der Schaftform zugänglich ist und wobei die Schaftform eine proximale Wandung aufweist, die die proximale Öffnung der Schaftform umlaufend begrenzt,

eine Kopfform zum Formen eines Kopfs und eines Halses des Spacers aus Knochenzementteig, wobei die Kopfform in ihrem Inneren einen Hohlraum aufweist, wobei der Hohlraum eine kugelflächenförmige Innenfläche zum Formen einer Gleitfläche des Kopfs des Spacers aufweist und der Hohlraum über eine distale Öffnung an einer distalen Seite der Kopfform zugänglich ist, wobei die Kopfform eine distale Wandung aufweist, die die distale Öffnung der Kopfform umlaufend begrenzt, und aufweisend
einen Metallkern, wobei der Metallkern einen Schaftteil, einen Kopfteil und einen Steg aufweist, wobei der Steg vom Metallkern absteht, wobei der Steg zwischen dem Schaftteil und dem Kopfteil angeordnet ist und wobei der Steg eine proximale Oberfläche und eine distale Oberfläche aufweist,
wobei die Schaftform und das Schaftteil des Metallkerns derart geformt sind, dass das Schaftteil in dem Innenraum der Schaftform angeordnet ist, wenn die proximale Wandung der Schaftform an der distalen Oberfläche des Stegs anliegt, und
wobei die Kopfform und das Kopfteil des Metallkerns derart geformt sind, dass das Kopfteil in dem Hohlraum der Kopfform angeordnet ist, wenn die distale Wandung der Kopfform an der proximalen Oberfläche des Stegs anliegt.

Erfindungsgemäß bevorzugt kann vorgesehen sein, dass die proximale Wandung der Schaftform an der distalen Oberfläche des Stegs flächenbündig anlegbar ist und/oder die distale Wandung der Kopfform an der proximalen Oberfläche des Stegs flächenbündig anlegbar ist.

Bevorzugt sind die Schaftform und das Schaftteil des Metallkerns derart geformt, dass das Schaftteil vollständig in dem Innenraum der Schaftform angeordnet ist, wenn die proximale Wandung der Schaftform an der distalen Oberfläche des Stegs anliegt, insbesondere flächenbündig an der distalen Oberfläche des Stegs anliegt.

Ebenfalls bevorzugt sind die Kopfform und das Kopfteil des Metallkerns derart geformt, dass das Kopfteil vollständig in dem Hohlraum der Kopfform angeordnet ist, wenn die distale Wandung der Kopfform an der proximalen Oberfläche des Stegs anliegt, insbesondere flächenbündig an der proximalen Oberfläche des Stegs anliegt.

Bevorzugt besteht der Metallkern aus einem biokompatiblen Metall.

Der Steg kann durchgehend umlaufend geformt sein oder auch eine oder mehrere Durchbrechungen aufweisen.

Besonders bevorzugt ist die Vorrichtung eine Vorrichtung zum Herstellen eines Hüft-Spacers.

Es kann vorgesehen sein, dass der Metallkern vollständig oder bis auf den Steg innerhalb der Schaftform und der Kopfform anzuordnen ist.

Bevorzugt steht der Steg transversal oder radial vom Metallkern ab.

Die proximale Oberfläche des Stegs begrenzt eine mögliche Verschiebung der Kopfform in distaler Richtung des Metallkerns und die distale Oberfläche des Stegs begrenzt eine mögliche Verschiebung der Schaftform in proximaler Richtung des Metallkerns.

In einer bevorzugten Ausgestaltungsform ist der Steg als ein den Metallkern umlaufender Kragen ausgebildet, wobei der Kragen den Metallkern ringförmig umgibt oder als elliptischer Kragen geneigt gegen einen Schaft des Metallkerns angeordnet ist. Ein umlaufender Kragen hat den Vorteil, dass der Anschlag der Kopfform und der Schaftform sicher gewährleistet wird.

Die Richtungsangaben proximal und distal und transversal werden in Übereinstimmung mit den anatomischen Richtungsbezeichnungen verstanden. Distal bedeutet dabei von der Körpermitte weg und proximal bedeutet zur Körpermitte hin. Bezüglich des Spacers und der Vorrichtung zum Herstellen des Spacers werden die Richtungsbezeichnungen dabei so verwendet, wie dies für den Spacer beziehungsweise den damit hergestellten Spacer im beim Patienten eingesetzten Zustand korrekt wäre.

Unter einem Zylinder und einer Zylindergeometrie wird vorliegend ein allgemeiner Zylinder verstanden. Ein allgemeiner Zylinder entsteht durch die lineare Verschiebung einer Grundfläche, hier der Grundfläche, die von dem umlaufenden Rand der Kavität begrenzt ist. Bei einem geraden Zylinder erfolgt die Verschiebung senkrecht zu dieser Grundfläche, bei einem schiefen Zylinder in einem von 90° abweichenden Winkel.

Es kann erfindungsgemäß vorgesehen sein, dass der Metallkern aus dem Schaftteil, dem Kopfteil und dem Steg besteht und/oder der Metallkern einteilig ist.

Hierdurch kann der Metallkern im Inneren des Spacers besser die auftretenden Kräfte aufnehmen und die Vorrichtung ist einfacher im Aufbau und der Anwendung.

Es kann auch vorgesehen sein, dass die Schaftform und/oder die Kopfform aus einem Kunststoff besteht oder bestehen, bevorzugt aus zumindest einer Kunststofffolie oder aus spritzgegossenem Kunststoff besteht oder bestehen.

Hierdurch wird die Vorrichtung kostengünstig im Aufbau und leicht anwendbar. Zudem können die Schaftform und die Kopfform nach der Anwendung so leicht, kostengünstig und vor allem hygienisch durch Verbrennen entsorgt werden.

Ferner kann vorgesehen sein, dass das Schaftteil in dem Innenraum der Schaftform von Innenwänden der Schaftform, die den Innenraum begrenzen, beabstandet ist, wenn die proximale Wandung der Schaftform an der distalen Oberfläche des Stegs anliegt, insbesondere flächenbündig an der distalen Oberfläche des Stegs anliegt, und/oder das Kopfteil in dem Hohlraum der Kopfform von Innenwänden der Kopfform, die den Hohlraum begrenzen, beabstandet ist, wenn die distale Wandung der Kopfform an der proximalen Oberfläche des Stegs anliegt, insbesondere flächenbündig an der proximalen Oberfläche des Stegs anliegt.

Hierdurch gelingt es, den Metallkern zentriert in der Kopfform beziehungsweise in der Schaftform anzuordnen, so dass dieser von einer gleichmäßig dicken Schicht des Knochenzementteigs umgeben ist.

Des Weiteren kann vorgesehen sein, dass die Kopfform eine Halsform zum Formen eines den Kopf und den Schaft verbindenden Halses des Spacers aus Knochenzementteig aufweist, wobei die Halsform rohrförmig oder hohlzylinderförmig ist, wobei die distale Öffnung und die distale Wandung der Kopfform beides Teile der Halsform sind und die kugelflächenförmige Innenfläche des Hohlraums kein Teil der Halsform ist, wobei vorzugsweise die Kopfform und das Kopfteil des Metallkerns derart geformt sind, dass das Kopfteil über die Halsform hinaus in den Hohlraum der Kopfform ragt, wenn die distale Wandung an der proximalen Oberfläche des Stegs anliegt.

Durch die Halsform kann die Kopfform und damit die Vorrichtung variabel zur Herstellung unterschiedlich geformter Spacer mit unterschiedlichen Halslängen genutzt werden. Die Kürzung der Halsform der Kopfform kann in einfacher Weise mit einer Schere, einem Messer, einem Skalpell oder einer Säge erfolgen.

Dabei kann vorgesehen sein, dass die Länge der Halsform durch Kürzen der Halsform einstellbar ist, wobei vorzugsweise Sollschnittstellen oder Sollreißstellen an der Halsform angeordnet sind und/oder eine Skalierung außen an der Halsform angeordnet ist.

Hierdurch kann die Vorrichtung besonders schnell und unkompliziert eingesetzt werden.

Ferner kann vorgesehen sein, dass die proximale Oberfläche des Stegs eine Verschiebung des Metallkerns in der Kopfform, insbesondere in der Halsform, begrenzt und/oder die distale Oberfläche des Stegs eine Verschiebung des Metallkerns in der Schaftform begrenzt.

Auf diese Weise wird durch den Steg ein Anschlag für die Kopfform und/oder die Schaftform erzeugt, so dass die Vorrichtung auf einfache Weise einsetzbar und zusammensetzbar ist.

Gemäß einer bevorzugten Ausführung kann vorgesehen sein, dass am Kopfteil des Metallkerns Zentriereinrichtungen angeordnet sind, wobei die Zentriereinrichtungen von der Oberfläche des Kopfteils abstehen, wobei die Zentriereinrichtungen Innenwände der Kopfform von dem Kopfteil beabstanden, wenn das Schafteil des Metallkerns in den Hohlraum der Kopfform eingeschoben ist, insbesondere die Halsform der Kopfform von dem Kopfteil beabstanden, wenn das Schafteil des Metallkerns in den Hohlraum der Kopfform eingeschoben ist, wobei vorzugsweise die Zentriereinrichtungen Zentrierfinnen sind und das Kopfteil des Metallkerns entlang der Zentrierfinnen axial in der Kopfform, insbesondere in der Halsform, verschiebbar ist, und/oder am Schaftteil des Metallkerns Abstandhalter angeordnet sind, wobei die Abstandhalter von der Oberfläche des Schaftteils abstehen, wobei die Abstandhalter Innenwände der Schaftform vom Schaftteil des Metallkerns beabstanden, wenn das Schaftteil des Metallkerns in den Innenraum der Schaftform eingeschoben ist.

Hierdurch wird erreicht, dass das Kopfteil des Metallkerns in der Kopfform und/oder das Schaftteil des Metallkerns in der Schaftform zentriert werden und allseitig von Knochenzementteig umgeben sein können beziehungsweise umflossen werden können. Dies vereinfacht zudem auch das Zusammensetzen der Teile der Vorrichtung während der Herstellung der Spacer. Durch die Abstandhalter wird der Abstand des Metallkerns von der Außenseite des zu gießenden Schafts gewährleistet.

Die proximale Oberfläche des Stegs begrenzt die axiale Verschiebung der Kopfform parallel zu den Zentriereinrichtungen beziehungsweise zu den Zentrierfinnen in distaler Richtung des Metallkerns und die distale Oberfläche des Stegs begrenzt eine Verschiebung der Schaftform in proximaler Richtung des Metallkerns.

Die Zentriereinrichtungen beziehungsweise die Zentrierfinnen liegen insbesondere am inneren Rand der hohlzylinderförmigen Halsform der Kopfform an und zentrieren die Kopfform so, dass die Achse der Kopfform auf der Achse des proximalen Metallkerns liegt. Die hohlzylinderförmige Halsform der Kopfform kann manuell entsprechend den anatomischen Erfordernissen des Patienten gekürzt werden, so dass der Offset individuell eingestellt werden kann. Der Vorteil besteht darin, dass dadurch der Spacerhals und der Spacerkopf einstückig sind und dadurch der Verbund beider Teile des Hüftspacer sicher gewährleistet wird. Montageschritte zur Befestigung des Spacerkopfs am Hals beziehungsweise am Schaft des Hüftspacers wie bei den Patentschriften US 7 789 646 B2, US 8 480 389 B2 und US 8 801 983 B2 sind nicht notwendig.

Bevorzugt sind die Zentriereinrichtungen und/oder die Abstandhalter einteilig mit dem Metallkern ausgeführt. Dies vereinfacht die Vorrichtung weiter.

Des Weiteren kann vorgesehen sein, dass der Außenumfang des Stegs größer ist als die proximale Öffnung der Schaftform und/oder als die distale Öffnung der Kopfform.

Hiermit kann verhindert werden, dass der Steg ungewollt und zu tief durch die distale Öffnung der Kopfform in den Hohlraum der Kopfform oder durch die proximale Öffnung der Schaftform in den Innenraum der Schaftform geschoben werden kann. Zudem wird dadurch gewährleistet, dass der distale Rand (die distale Wandung) der Kopfform sicher in seiner axialen Bewegung gegen den Metallkern begrenzt wird.

Auch kann vorgesehen sein, dass wenigstens eine Entlüftungsöffnung in der Schaftform und/oder der Kopfform angeordnet ist, wobei die wenigstens eine Entlüftungsöffnung den Innenraum der Schaftform und/oder den Hohlraum der Kopfform gasdurchlässig mit der Umgebung der Vorrichtung verbindet, wobei vorzugsweise die wenigstens eine Entlüftungsöffnung für Gase durchlässig ist und für Knochenzementteig undurchlässig ist, insbesondere für Polymethylmethacrylat-Knochenzementteig undurchlässig ist.

Hierdurch kann erreicht werden, dass Lufteinschlüsse besser aus dem Innenraum der Schaftform beziehungsweise aus dem Hohlraum der Kopfform entweichen können. Dadurch werden unerwünschte Fehlstellen an der Oberfläche des Spacers verhindert.

Es kann ferner vorgesehen sein, dass die Vorrichtung ein in zumindest einem keimdichten Zementbehälter verpacktes Zementpulver und eine in zumindest einem keimdichten und flüssigkeitsdichten Monomerflüssigkeitsbehälter verpackte Monomerflüssigkeit aufweist.

Hierdurch wird die Vorrichtung weiter komplettiert. Die Vorrichtung ist dann gleich dazu bereit, einen Spacer herzustellen.

Dabei kann vorgesehen sein, dass die Vorrichtung zumindest eine Knochenzementkartusche zum Mischen des Zementpulvers mit der Monomerflüssigkeit und zum Austragen von gemischtem Knochenzementteig aus der Knochenzementkartusche heraus aufweist, bevorzugt eine Knochenzementkartusche zum Mischen von Polymethylmethacrylat-Knochenzement-Ausgangskomponenten und zum Austragen von gemischtem Polymethylmethacrylat-Knochenzementteig aus der Knochenzementkartusche heraus aufweist, wobei besonders bevorzugt die zumindest eine Knochenzementkartusche den zumindest einen Zementbehälter aufweist und den zumindest einen Monomerflüssigkeitsbehälter enthält, wobei der zumindest eine Zementbehälter und der zumindest eine Monomerflüssigkeitsbehälter in voneinander getrennten Bereichen angeordnet sind.

Hierdurch kann der mit der Knochenzementkartusche erzeugte Knochenzementteig bequem in die Schaftform und die Kopfform eingefüllt werden.

Die der Erfindung zugrundeliegenden Aufgaben werden auch gelöst durch ein Set zum Herstellen unterschiedlicher Spacer durch Aushärten von Knochenzementteig, wobei die Spacer dazu vorgesehen sind, im medizinischen Bereich ein Gelenk oder einen Teil eines Gelenks umfassend eine artikulierende Oberfläche eines Kopfs des Gelenks temporär zu ersetzen, insbesondere eines Hüftgelenks oder eines Schultergelenks temporär zu ersetzen, das Set aufweisend mindestens eine oben beschriebene erfindungsgemäße Vorrichtung, wobei das Set zumindest zwei Kopfformen mit unterschiedlichem Durchmesser der kugelflächenförmigen Innenflächen aufweist und/oder zumindest zwei Schaftformen mit in distaler Richtung unterschiedlich lang gestreckten Hohlräumen oder unterschiedlichen Innendurchmessern der Hohlräume aufweist.

Hierdurch wird ein variables Set bereitgestellt, mit dem unterschiedlich geformte Spacer hergestellt werden können, die zu einer Vielzahl von unterschiedlichen Behandlungssituationen passen.

Besonders bevorzugt ist das Set ein Set zum Herstellen eines Hüft-Spacers.

Bei dem Set kann vorgesehen sein, dass das Set zumindest zwei Metallkerne mit unterschiedlicher Länge des Kopfteils und/oder des Schaftteils aufweist.

Hierdurch wird das Set noch variabler.

Die der vorliegenden Erfindung zugrundeliegenden Aufgaben werden auch gelöst durch ein Verfahren zur Herstellung eines Spacers zum temporären Ersetzen eines Gelenks oder eines Teils eines Gelenks, insbesondere eines Hüftgelenks oder eines Schultergelenks, umfassend eine artikulierende Oberfläche des Gelenks, wobei das Verfahren mit einer erfindungsgemäßen Vorrichtung oder mit einem erfindungsgemäßen Set durchgeführt wird, das Verfahren aufweisend die folgenden Schritte:
A) Einfüllen eines Knochenzementteigs in den Innenraum der Schaftform und Einfüllen eines Knochenzementteigs in den Hohlraum der Kopfform;
B) Einschieben des Kopfteils des Metallkerns in den mit Knochenzementteig gefüllten Hohlraum der Kopfform unter Verdrängung des darin enthaltenen Knochenzementteigs, bis die distale Wandung der Kopfform an der proximalen Oberfläche des Stegs des Metallkerns anliegt, und Einschieben des Schaftteils des Metallkerns in den mit Knochenzementteig gefüllten Innenraum der Schaftform unter Verdrängung des darin enthaltenen Knochenzementteigs, bis die proximale Wandung der Schaftform an der distalen Oberfläche des Stegs des Metallkerns anliegt;
C) Aushärten des Knochenzementteigs in der Kopfform und der Schaftform; und
D) Entnehmen des so geformten und ausgehärteten Spacers aus der Kopfform und der Schaftform.

Besonders bevorzugt ist das Verfahren ein Verfahren zum Herstellen eines Hüftspacers.

Das Verfahren umfasst keinen Schritt zur therapeutischen oder medizinischen Behandlung eines menschlichen oder tierischen Körpers. Das Verfahren betrifft lediglich die Herstellung eines Spacers, der anschließend medizinisch eingesetzt werden kann. Letzteres erfolgt aber nicht im Rahmen des erfindungsgemäßen Verfahrens.

Der Schritte C) erfolgt nach den Schritten A) und B) und der Schritt D) erfolgt nach Schritt C). Die Schritte A) und B) können nacheinander aber auch parallel erfolgen.

Es kann vorgesehen sein, dass vor Schritt A) eine Halsform der Kopfform entsprechend einer gewünschten Länge des Halses des herzustellenden Spacers gekürzt wird, eine Kopfform mit einem passenden Innendurchmesser der kugelflächenförmigen Innenfläche passend zur gewünschten Form des Kopf des herzustellenden Spacers ausgewählt wird, eine Schaftform mit einem zur gewünschten Form des Schafts des herzustellenden Spacers passenden Innenraum ausgewählt wird und/oder ein Metallkern mit zu der gewünschten Form des Schafts des herzustellenden Spacers passenden Abmessungen ausgewählt wird.

Hierdurch wird ein variables Verfahren bereitgestellt, mit dem unterschiedlich geformte Spacer hergestellt werden können, die zu einer Vielzahl von unterschiedlichen Behandlungssituationen passen.

Des Weiteren kann vorgesehen sein, dass vor Schritt A) der homogener Knochenzementteig durch Mischen einer Monomerflüssigkeit und einem Zementpulver hergestellt wird, wobei bevorzugt in Schritt A) der gemischte Knochenzementteig mit einer Knochenzementkartusche in den Innenraum der Schaftform und in den Hohlraum der Kopfform gepresst wird, wobei besonders bevorzugt dabei die Luft aus dem Innenraum der Schaftform und aus dem Hohlraum der Kopfform verdrängt wird.

Hiermit wird das Verfahren weiter komplettiert.

Auch kann vorgesehen sein, dass in Schritt B) das Kopfteil des Metallkerns mit Hilfe von Zentriereinrichtungen, insbesondere mit Hilfe von Zentrierfinnen, die von der Oberfläche des Kopfteils abstehen und die beim Einschieben des Kopfteils in den Hohlraum der Kopfform an Innenwänden des Hohlraums der Kopfform anliegen, in der Kopfform zentriert werden, und/oder in Schritt B) das Schaftteil des Metallkerns mit Hilfe von Abstandhaltern, die von der Oberfläche des Schaftteils abstehen und die beim Einschieben des Schaftteils in den Innenraum der Schaftform an Innenwänden des Innenraums der Schaftform anliegen, in der Schaftform zentriert werden.

Hierdurch wird erreicht, dass das Kopfteil des Metallkerns in der Kopfform und/oder das Schaftteil des Metallkerns in der Schaftform zentriert werden und allseitig von Knochenzementteig umgeben sein können beziehungsweise umflossen werden können. Dies vereinfacht zudem auch das Zusammensetzen der Teile der Vorrichtung während der Herstellung der Spacer.

Der Erfindung liegt die überraschende Erkenntnis zugrunde, dass eine Kopfform und eine Schaftform mit einem Knochenzementteig im Überschuss gefüllt werden können und diese auf einen Metallkern mit einem geeigneten Steg als Anschlag aufgeschoben werden können, so dass der überschüssige Knochenzementteig aus der zusammengesetzten Gießform gedrückt wird und der Metallkern bereichsweise in dem Knochenzementteig in der zusammengesetzten Gießform eingeschlossen wird. Hierdurch kann erfindungsgemäß vermieden werden, dass der Knochenzementteig durch die Zwischenräume zwischen dem Metallkern und den Innenwandungen der Kopfform und der Schaftform hinein gepresst werden muss, was insbesondere bei hochviskosen Knochenzementteigen einen erheblichen Druck und Kraftaufwand erfordert. Dadurch wiederum können die Kopfform und die Schaftform kostengünstig aus einfachen Kunststoffen gefertigt werden, und können daher beispielsweise durch Kunststoffspritzgießen oder durch Tiefziehen von Kunststofffolien in einfacher Weise hergestellt werden. Der aus der Kopfform und der Schaftform ausgetretene überschüssige Knochenzementteig kann entfernt und entsorgt werden, bevor oder nachdem der Knochenzementteig in der Gießform aushärtet.

Die Schaftform und die Kopfform können erfindungsgemäß kostengünstig durch Spritzguss aus Kunststoff hergestellt werden oder sogar auch aus einer tiefgezogenen Kunststofffolie hergestellt werden, da sie selbst beim Einfüllen eines hochviskosen Knochenzementteigs keine sehr großen Kräfte aufnehmen müssen. Dies ist möglich, weil erfindungsgemäß der Knochenzementteig in die beiden Teile der Gießform gefüllt wird, ohne dass der Metallkern bereits darin angeordnet ist und deswegen umflossen werden müsste. Die Anordnung des Metallkerns in der Schaftform und in der Kopfform im Anschluss an das Einfüllen des Knochenzementteigs ist dadurch möglich, dass die Schaftform und die Kopfform an den Steg des Metallkerns angelegt werden und so passend zum Metallkern und passend zueinander angeordnet und ausgerichtet werden. Dadurch müssen die Schaftform und die Kopfform nicht aufwendig und Druck-stabil aneinandergeschraubt beziehungsweise aneinander befestigt werden.

Ein weiterer besonderer Vorteil der vorliegenden Erfindung besteht darin, dass mit der Kopfform ein zylindrischer Bereich, der als Halsform bezeichnet werden kann und der zum Ausformen eines Halses des Spacers, der den Kopf des Spacers mit dem Schaft des Spacers verbindet, variabel gestaltet werden kann, indem der zylindrische Bereich manuell zu kürzen ist, je nach der anatomischen Anforderung an den jeweiligen Patienten. Zur Herstellung von Spacern mit unterschiedlichen Kopfdurchmessern können unterschiedlich geformte Kopfformen verwendet und ausgewählt werden. Auch die anderen Abmessungen können durch unterschiedlich geformte Schaftformen und unterschiedlich geformte Metallkerne mit einem erfindungsgemäßen Set angepasst werden.

Die erfindungsgemäße Vorrichtung wird in der Weise verwendet, dass zuerst Knochenzementteig in die Kopfform retrograd eingebracht wird. Dazu kann ein Mischsystem beziehungsweise Kartuschensystem als Knochenzementapplikator mit einer Kartusche und eine manuell zu bedienende Auspresspressvorrichtung benutzt werden. Dann wird der proximale Kopfteil des Metallkerns in die mit Knochenzementteig gefüllte Kopfform gedrückt. Der proximale Kopfteil des Metallkerns dringt in den Knochenzementteig die distale Wandung als der Rand der Kopfform beziehungsweise der Rand des hohlzylinderförmigen Halsteils der Kopfform auf die proximale Oberfläche des Stegs trifft. Der Steg bewirkt eine Begrenzung der axialen Bewegung der Kopfform. Anschließend wird die Schaftform mit Knochenzementteig retrograd befüllt. Danach wird das Schaftteil des Metallkern in die mit Knochenzementteig befüllte Schaftform eingedrückt, bis die proximale Wandung als Rand der Schaftform auf der distalen Oberfläche des Stegs anschlägt. Der überschüssige, verdrängte Knochenzementteig quillt aus der Öffnung der Schaftform aus und wird manuell entfernt. Anschließend lässt man den Knochenzementteig aushärten. Danach wird die Kopfform durch Aufschneiden entfernt und die Schaftform wird vom ausgehärteten Schaft des gebildeten Spacers abgezogen. Die Fertigung des Spacers mit der erfindungsgemäßen Vorrichtung kann auch so erfolgen, dass zuerst die Schaftform und danach die Kopfform mit Knochenzementteig befüllt wird. Der Metallkern wird auch hierbei in die mit Knochenzementteig befüllten Formen gedrückt.

Der erfindungsgemäßen Vorrichtung liegt der überraschende Befund zu Grunde, dass ein Metallkern in eine mit Knochenzementteig gefüllte Gießform mit geringerer Kraft gedrückt werden kann, als Knochenzementteig um einen in der Gießform angeordneten Metallkern durch Presskraft, erzeugt durch manuell betätigbare Auspressvorrichtungen, gedrückt werden kann, wie es bei den bekannten Gießvorrichtungen gemäß den Patentschriften US 7 789 646 B2, US 8 480 389 B2 und US 8 801 983 B2 notwendig ist.

Dadurch ist es möglich, auch mit hochviskosem Knochenzementteig ohne Einsatz von hoher Presskraft innerhalb weniger Minuten Spacer herzustellen. Druckfeste Gießformen sind nicht erforderlich. Die erfindungsgemäße Gießform wird annähernd druckfrei befüllt und kann daher aus dünnen Kunststofffolien oder spritzgegossenen Kunststoffkörpern gefertigt werden.

Eine beispielhafte erfindungsgemäße Vorrichtung kann zusammengesetzt sein aus
a) einem Metallkern, der aus einem Kopfteil und einen Schaftteil besteht, wobei das Kopfteil durch ein oder mehrere Stege, die an der Metallkernoberfläche angeordnet sind, vom Schaftteil getrennt ist, wobei der mindestens eine Steg eine proximale Seite (Oberfläche) und eine distale Seite (Oberfläche) besitzt,
b) einer aus Kunststoff gefertigten hohlen Spacerkopfform beziehungsweise Kopfform mit einer hohlzylinderförmigen Halsform,
c) einer aus Kunststoff gefertigten hohlen Schaftform, wobei
d) das Kopfteil des Metallkerns in einen Innenraum der Kopfform ragt, wenn die hohlzylinderförmige Halsform auf der proximalen Seite des mindestens einen Steges aufliegt und das Schaftteil des Metallkerns vollständig von der Schaftform umgeben wird, wenn das Schaftteil mit seiner Öffnung an der distalen Fläche des mindestens eines Steges aufliegt.

Ein beispielhaftes erfindungsgemäßes Set zur Herstellung von Spacern kann zusammengesetzt sein aus
a) einem Metallkern, der aus einem Kopfteil, einem Steg und einem Schaftteil besteht,
b) mindestens einer Schaftform, und
c) mindestens zwei Kopfformen mit unterschiedlichen Kopfdurchmessern, die in einem sterilisierbaren, keimdichten Packmittel angeordnet sind.

Das erfindungsgemäße Set bietet den Vorteil, dass mit einer Packungseinheit des Sets Spacer mit unterschiedlichen Kopfgrößen vom medizinischen Anwender hergestellt werden können. Dadurch entfällt die Notwendigkeit, separate Packungseinheiten für jede Kopfgröße des Spacers bereitzustellen.

Ein weiteres beispielhaftes erfindungsgemäßes Set zur Herstellung von Spacern kann zusammengesetzt sein aus
a) einem Zementpulver verpackt in einem keimdichten Behälter,
b) einer Monomerflüssigkeit verpackt in einem keimdichten, flüssigkeitsundurchlässigen Behälter,
c) einem Metallkern, das aus einem Kopfteil, mindestens einem Steg und einem Schaftteil besteht,
d) mindestens einer Schaftform, und
e) mindestens zwei Kopfformen mit unterschiedlichen Kopfdurchmessern, die in einem sterilisierbaren, keimdichten Packmittel angeordnet sind.

Solche Sets als sogenannter "Procedure-Pack" dem medizinischen Anwender zur Verfügung gestellt werden. Alle für die Herstellung von Hüftspacern erforderlichen Komponenten sind im Set enthalten.

Ein beispielhaftes erfindungsgemäßes Verfahren zur Herstellung von Spacern mit der erfindungsgemäßen Vorrichtung kann die folgenden nacheinander ablaufenden Schritte umfassen:
a) Vermischen eines Zementpulvers mit einer Monomerflüssigkeit bis ein homogener Knochenzementteig entstanden ist,
b) Einfüllen des Knochenzementteigs in die Kopfform,
c) Einfüllen des Knochenzementteigs in die Schaftform,
d) Einschieben des Kopfteils des Metallkerns in die mit Knochenzementteig gefüllte Kopfform unter Verdrängung des Knochenzementteigs bis der Rand der hohlzylinderförmigen Halsform der Kopfform auf der proximalen Oberfläche des Stegs anliegt,
e) Einschieben des Schaftteils des Metallkerns in die mit Knochenzementteig gefüllte Schaftform unter Verdrängung des Knochenzementteigs bis die proximale Öffnung der Schaftform auf der distalen Oberfläche des Stegs aufliegt,
f) Aushärten des Knochenzementteigs und
g) Entfernung der Kopfform und der Schaftform nach vollständiger Aushärtung des Knochenzementteigs.

Erfindungsgemäß kann vorgesehen sein, dass vor dem Schritt a) die Länge des hohlzylinderförmigen Halsform der Kopfform zur Einstellung des Offsets, das heißt zur Einstellung des Abstands zwischen dem Kopf und dem Schaft des Spacers, gekürzt wird. Dazu wird vom medizinischen Anwender mit einer Schere oder einer Säge die hohlzylinderförmige Halsform an der Kopfform soweit gekürzt, bis der gewünschte Offset, der Abstand vom Zentrum der Kugelform bis zur Längsachse des Metallkerns, erreicht ist.

Im Folgenden werden weitere Ausführungsbeispiele der Erfindung anhand von fünfundzwanzig schematisch dargestellten Figuren erläutert.

Dabei zeigt:
Figur 1: eine schematische perspektivische Teilquerschnittansicht einer zusammengesetzten, beispielhaften ersten erfindungsgemäßen Vorrichtung zum Herstellen eines Hüftgelenkspacers;
Figur 2: eine schematische perspektivische Außenansicht der zusammengesetzten ersten erfindungsgemäßen Vorrichtung nach Figur 1;
Figur 3: eine schematische perspektivische Teilquerschnittansicht der zusammengesetzten ersten erfindungsgemäßen Vorrichtung nach den Figuren 1 und 2;
Figur 4: eine schematische perspektivische Außenansicht auf die Teile der ersten erfindungsgemäßen Vorrichtung nach den Figuren 1 bis 3;
Figur 5: eine schematische Teilquerschnittansicht der zusammengesetzten ersten erfindungsgemäßen Vorrichtung;
Figur 6: eine schematische Querschnittansicht der ersten erfindungsgemäßen Vorrichtung mit eingefülltem Knochenzementteig;
Figur 7: eine schematische Seitenansicht eines mit der ersten erfindungsgemäßen Vorrichtung hergestellten Hüftspacers;
Figur 8: eine perspektivische Teilquerschnittansicht einer zusammengesetzten, beispielhaften zweiten erfindungsgemäßen Vorrichtung zum Herstellen eines Hüftgelenkspacers;
Figur 9: eine Seitenansicht der zusammengesetzten zweiten erfindungsgemäßen Vorrichtung;
Figur 10: eine Teilquerschnittansicht der zusammengesetzten zweiten erfindungsgemäßen Vorrichtung;
Figur 11: eine perspektivische Ansicht eines mit der zweiten erfindungsgemäßen Vorrichtung hergestellten Hüftspacers;
Figur 12: eine Teilquerschnittansicht der zusammengesetzten zweiten erfindungsgemäßen Vorrichtung mit gekürzter Halsform;
Figur 13: eine Teilquerschnittansicht einer zusammengesetzten dritten erfindungsgemäßen Vorrichtung mit kleinerer Kopfform;
Figur 14: eine Teilquerschnittansicht der dritten erfindungsgemäßen Vorrichtung beim Einfüllen eines Knochenzementteigs zur Herstellung des Spacers;
Figur 15: eine Teilquerschnittansicht der zweiten erfindungsgemäßen Vorrichtung beim Einschieben des Metallkerns in die mit Knochenzementteig gefüllte Kopfform zur Herstellung des Spacers;
Figur 16: eine Teilquerschnittansicht der zweiten erfindungsgemäßen Vorrichtung mit bis zum Anschlag vollständig in die mit Knochenzementteig gefüllte Kopfform eingeschobenem Metallkern zur Herstellung des Spacers;
Figur 17: eine Teilquerschnittansicht der zweiten erfindungsgemäßen Vorrichtung beim Einfüllen eines Knochenzementteigs zur Herstellung des Spacers;
Figur 18: eine Teilquerschnittansicht der zweiten erfindungsgemäßen Vorrichtung beim Einschieben des Metallkerns in die mit Knochenzementteig gefüllte Schaftform zur Herstellung des Spacers;
Figur 19: eine Teilquerschnittansicht der zweiten erfindungsgemäßen Vorrichtung mit bis zum Anschlag vollständig in die mit Knochenzementteig gefüllte Schaftform eingeschobenem Metallkern zur Herstellung des Spacers;
Figur 20: eine Teilquerschnittansicht der zusammengesetzten und mit Knochenzementteig gefüllten zweiten erfindungsgemäßen Vorrichtung während dem Aushärten des Knochenzementteigs;
Figur 21: eine perspektivische Teilquerschnittansicht auf die Teile eines ersten erfindungsgemäßen Sets mit einer beispielhaften vierten erfindungsgemäßen Vorrichtung mit zwei unterschiedlichen Kopfformen zum Herstellen eines Hüftgelenkspacers;
Figur 22: eine perspektivische Außenansicht auf die Teile des ersten erfindungsgemäßen Sets nach Figur 21;
Figur 23: eine schematische perspektivische Teilquerschnittansicht der zusammengesetzten vierten erfindungsgemäßen Vorrichtung des ersten erfindungsgemäßen Sets nach den Figuren 21 und 22;
Figur 24 A) bis H): eine schematische Querschnittansicht durch acht sich hinsichtlich der Kopfform unterscheidende Vorrichtungen zur Herstellung eines Hüftspacers, die als erfindungsgemäßes Set genutzt werden können; und
Figur 25 A) bis H): eine schematische Seitenansicht auf Spacer, die mit den Vorrichtungen nach Figur 24 A) bis H) hergestellt wurden.

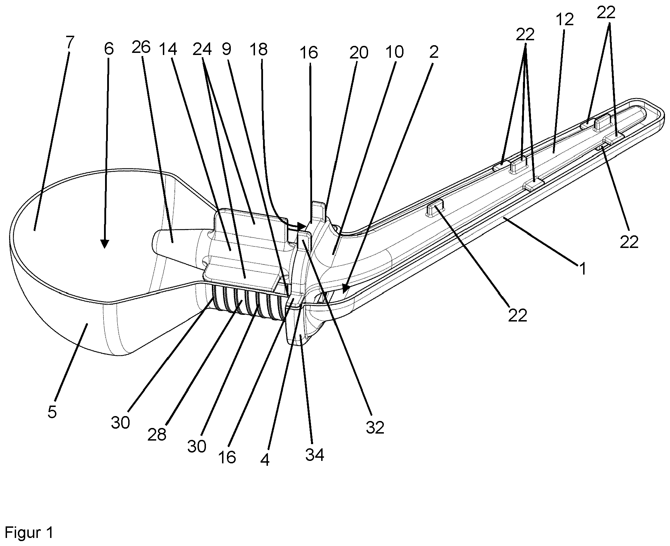

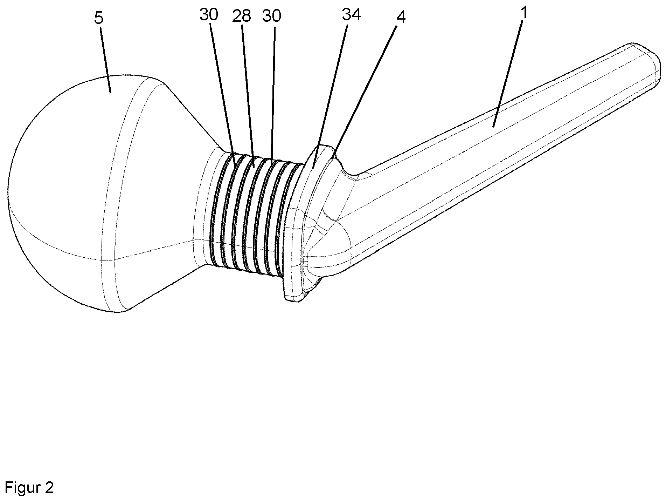

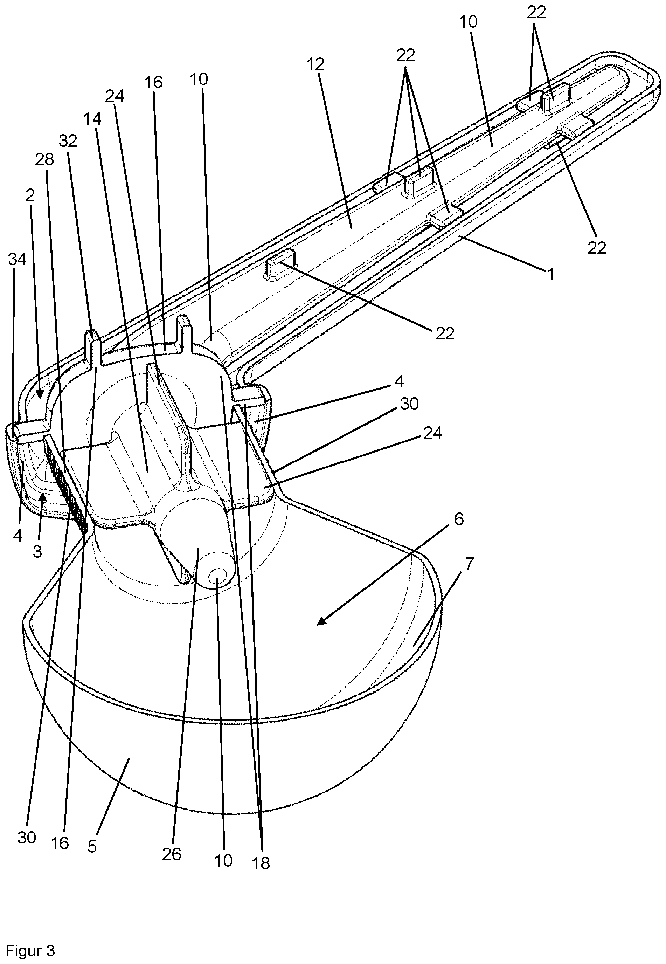

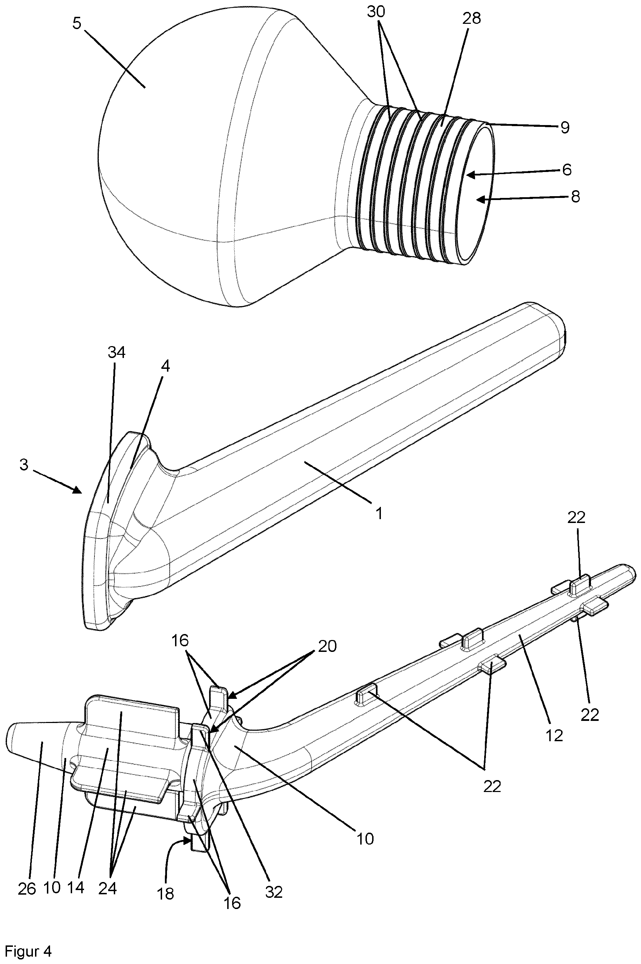

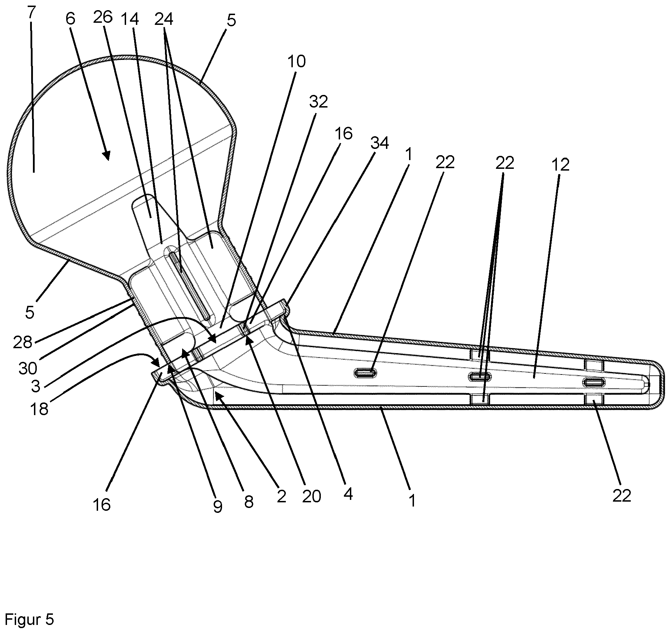

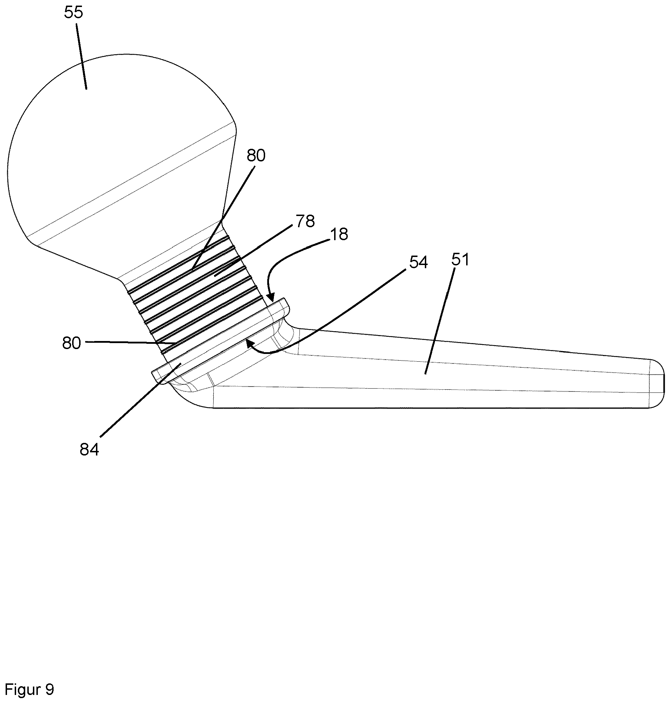

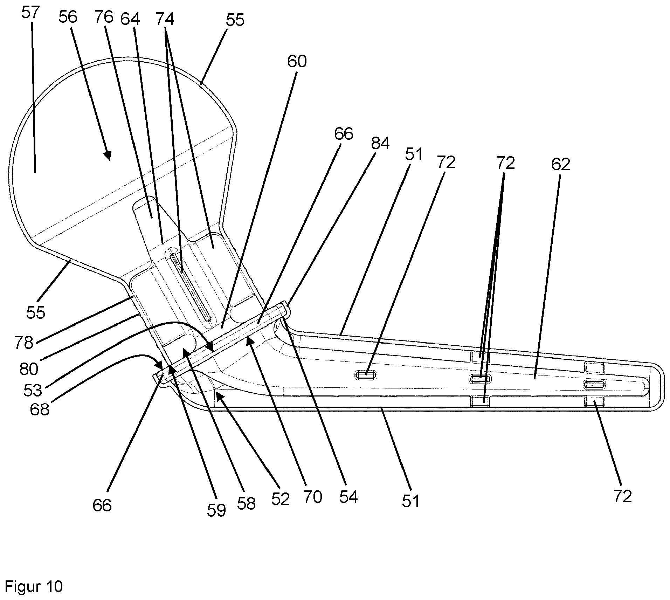

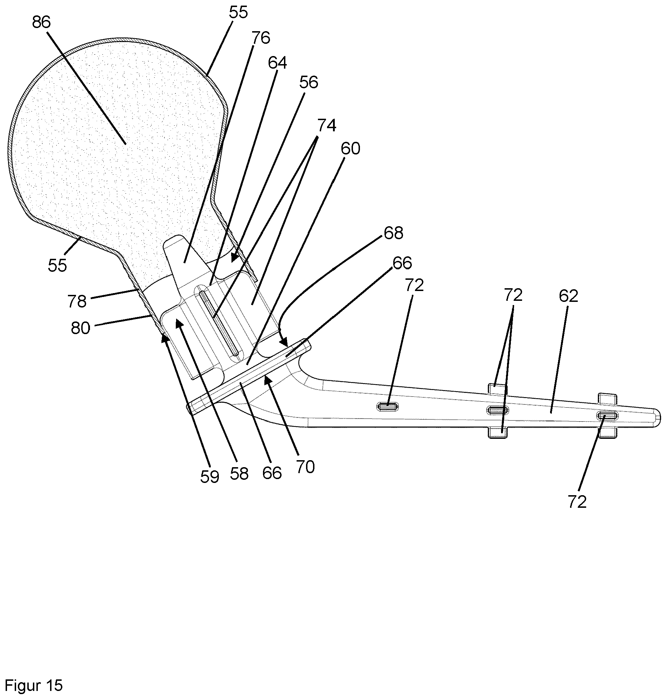

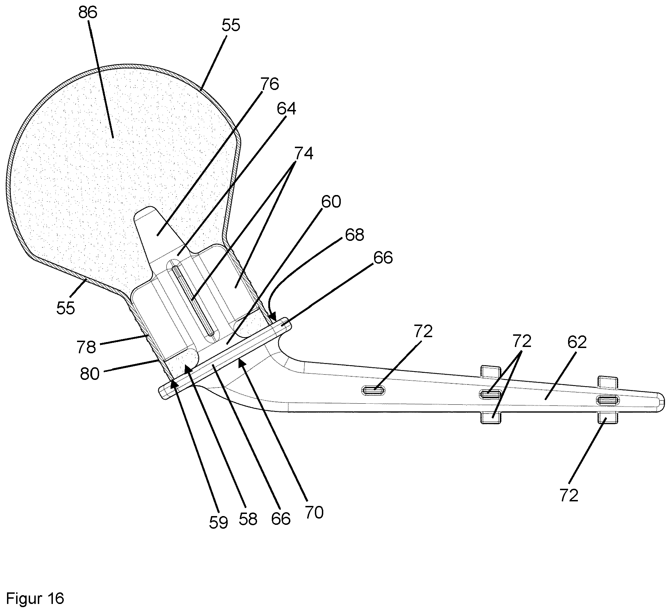

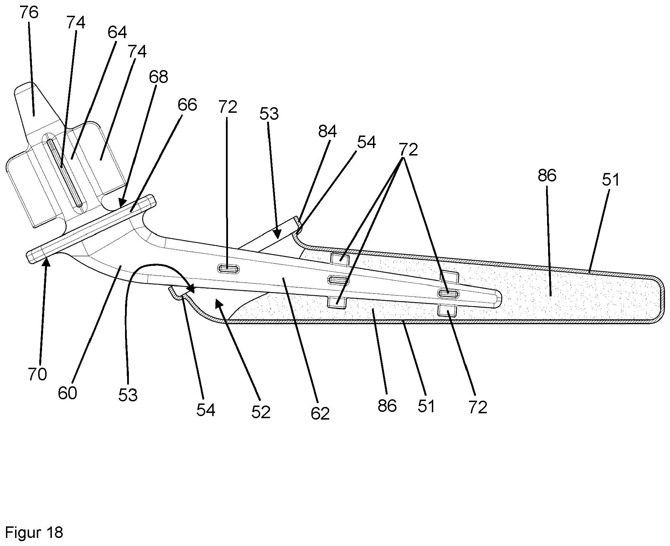

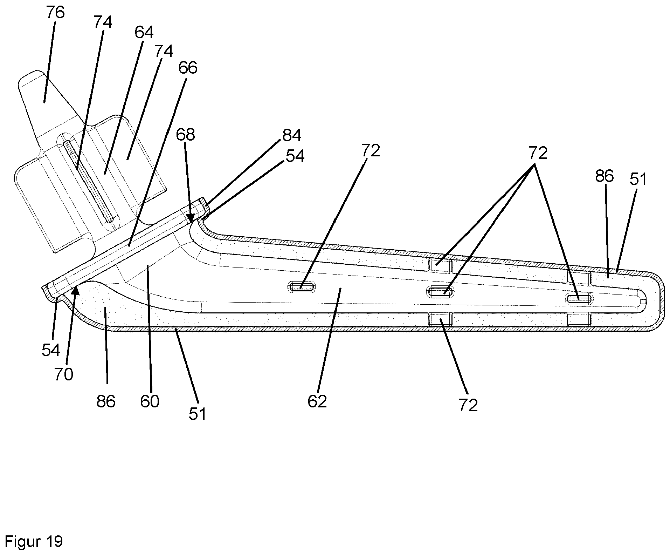

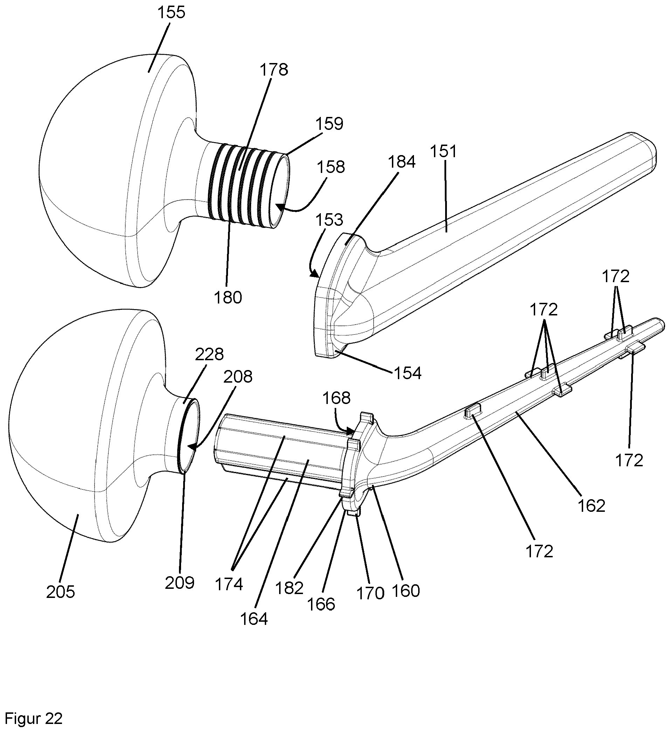

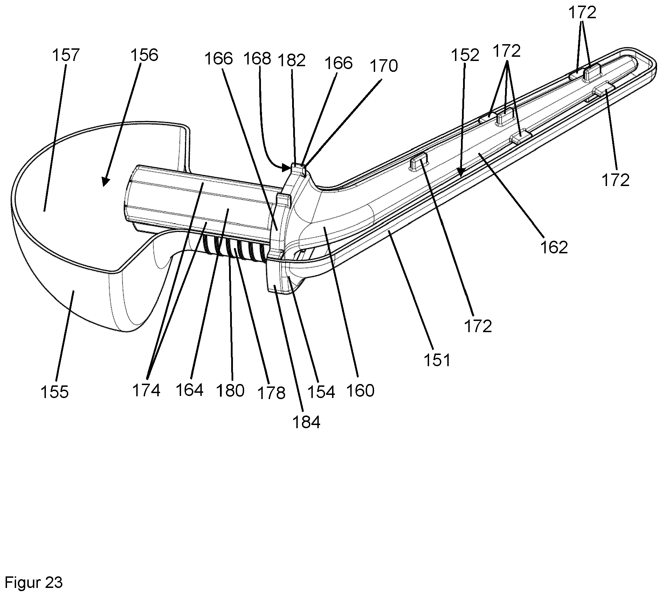

Die Figuren 1 bis 25 zeigen Vorrichtungen, Sets und deren Teile sowie Verfahrensabläufe, die zur Herstellung von Hüftspacern vorgesehen sind. Der Fachmann kann dies ohne weiteres auf Vorrichtungen, Sets und deren Teile sowie Verfahrensabläufe zur Herstellung von Schulterspacern übertragen.

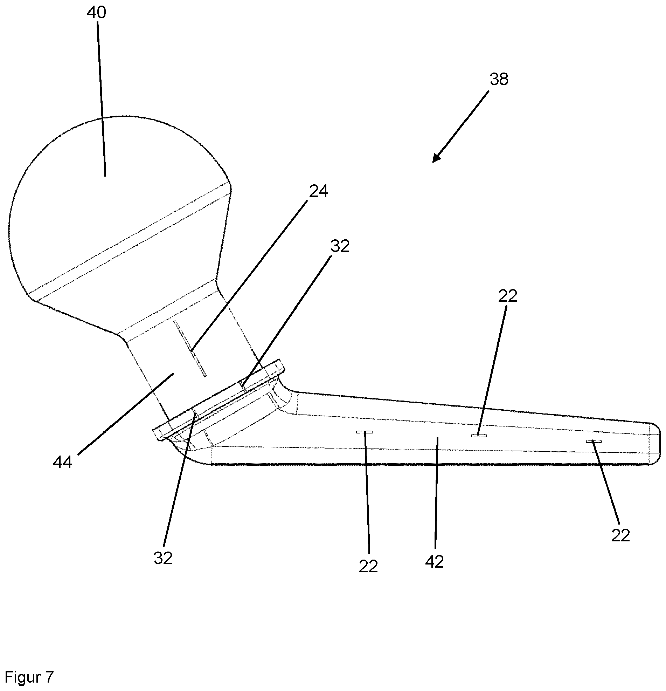

In den Figuren 1 bis 7 sind Abbildungen eines ersten Ausführungsbeispiels einer erfindungsgemäßen Vorrichtung zur Herstellung eines Hüftgelenkspacers, wie er in Figur 7 gezeigt ist, und Teile der Vorrichtung in verschiedenen Ansichten gezeigt.

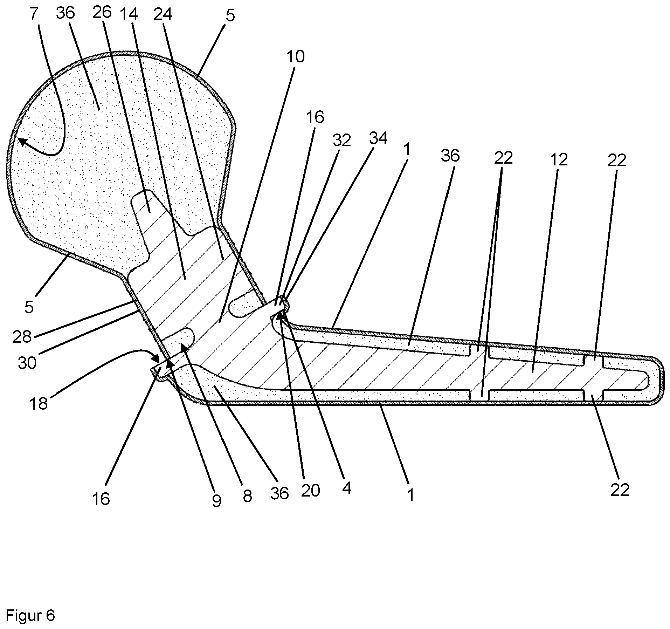

Die erste erfindungsgemäße Vorrichtung ist zur Herstellung eines Spacers 38 (siehe Figur 7) für ein Hüftgelenk geeignet und vorgesehen. Die Vorrichtung hat eine Schaftform 1 und eine Kopfform 5 und einen Metallkern 10. Die Schaftform 1 und die Kopfform 5 sind vorzugsweise jeweils einteilig aufgebaut. Die Schaftform 1 und die Kopfform 5 können gemeinsam eine zweiteilige Gießform zum Formen eines darin eingefüllten Knochenzementteigs 36 (siehe Figur 6) bilden. Hierzu härtet der Knochenzementteig 36 in der Gießform aus. In den Figuren 1, 3, 5 und 6 sind die Schaftform 1 und die Kopfform 5 geschnitten dargestellt, so dass der innere Aufbau der Vorrichtung erkennbar ist. Der Metallkern 10 ist nur in Figur 6 im Schnitt dargestellt und ansonsten ungeschnitten.

Die Schaftform 1 weist einen Innenraum 2 auf, in dem der Knochenzementteig 36 zu einem Schaft 42 des Spacers 38 formbar ist. Der Innenraum 2 ist über eine proximale Öffnung 3 zugänglich, so dass der Knochenzementteig 36 durch die proximale Öffnung 3 der Schaftform 1 in den Innenraum 2 einfüllbar ist und der Metallkern 10 teilweise durch die proximale Öffnung 3 in den Innenraum 2 der Schaftform 1 einschiebbar ist. Um die proximale Öffnung 3 herum weist die Schaftform 1 eine proximale Wandung 4 auf, die die proximale Öffnung 3 begrenzt und umschließt.

Die Kopfform 5 weist einen Hohlraum 6 auf, in dem der Knochenzementteig 36 zu einem Kopf 40 des Spacers 38 formbar ist. Der Hohlraum 6 weist an einer proximalen Seite des Hohlraums 6 eine kugelflächenförmige Innenfläche 7 auf, die zum Ausformen der eigentlichen Gelenkfläche des Spacers 38 dient. Dementsprechend formt die kugelflächenförmige Innenfläche 7 des Hohlraums 6 die Hüftgelenkoberfläche des Spacers 38, die in einer Hüftpfanne gleiten soll. Der Hohlraum 6 ist über eine distale Öffnung 8 zugänglich, so dass der Knochenzementteig 36 durch die distale Öffnung 8 der Kopfform 5 in den Hohlraum 6 einfüllbar ist und der Metallkern 10 teilweise durch die distale Öffnung 8 in den Hohlraum 6 der Kopfform 5 einschiebbar ist. Um die distale Öffnung 8 herum weist die Kopfform 5 eine distale Wandung 9 auf, die die distale Öffnung 8 begrenzt und umschließt.

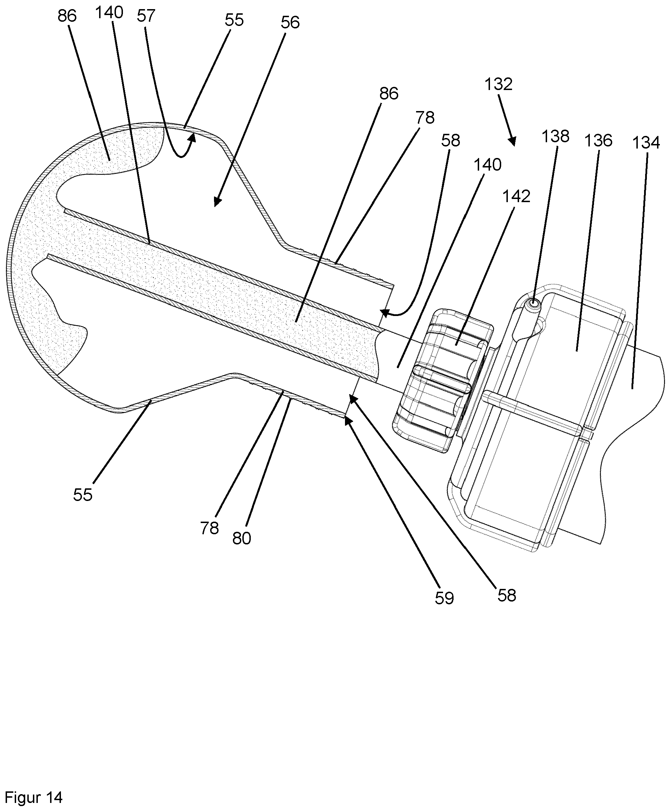

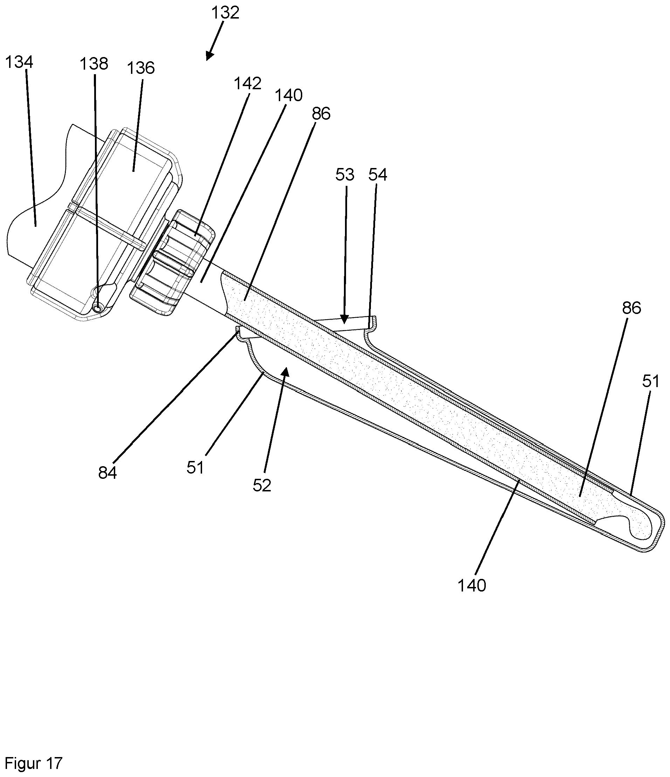

Der Knochenzementteig 36 kann vor dem Einfüllen aus einer Monomerflüssigkeit und einem Zementpulver (nicht gezeigt) gemischt worden sein, wobei dem Knochenzementteig 36 vorzugsweise auch zumindest ein Antibiotikum und/oder zumindest ein Antimykotikum beigemischt werden können. Der Knochenzementteig 36 kann über einen Knochenzementapplikator 132, wie er in den Figuren 14 und 17 zum zweiten Ausführungsbeispiel gezeigt ist, in die Schaftform 1 und in die Kopfform 5 gefüllt werden. Der Metallkern 10 kann aus einem biokompatiblen Metall, wie beispielsweise Edelstahl oder Titan bestehen. Der Metallkern 10 dient der Armierung und damit der mechanischen Stabilisierung des Spacers 38. Der Metallkern 10 hat ein Schaftteil 12, ein Kopfteil 14 und einen Steg 16, der zwischen dem Schaftteil 12 und dem Kopfteil 14 angeordnet ist. Vorzugsweise ist der Metallkern 10 einteilig. Das Schaftteil 12 und das Kopfteil 14 können über den Steg 16 miteinander verbunden sein. Der Steg 16 kann in radialer Richtung von den Achsen des Kopfteils 14 und des Schaftteils 12 abstehen. Der vorspringende Steg 16 weist eine proximale Oberfläche 18 und eine gegenüberliegende distale Oberfläche 20 auf.

Die proximale Wandung 4 der Schaftform 1 ist passend zu der distalen Oberfläche 20 des Stegs 16 geformt, so dass die Schaftform 1 zumindest bereichsweise flächenbündig and die distale Oberfläche 20 des Stegs 16 anlegbar ist. Dies erlaubt eine eindeutige Positionierung der Schaftform 1 an den Steg 16 des Metallkerns 10. In gleicher Weise ist die distale Wandung 9 der Kopfform 5 passend zu der proximalen Oberfläche 18 des Stegs 16 geformt, so dass die Kopfform 5 zumindest bereichsweise flächenbündig an die proximale Oberfläche 18 des Stegs 16 anlegbar ist. Dies erlaubt eine eindeutige Positionierung der Kopfform 5 an den Steg 16 des Metallkerns 10.

Am Schaftteil 12 können mehrere vorspringende Abstandhalter 22 mit länglicher Form angeordnet sein, die die Positionierung und Ausrichtung des Schaftteils 12 in der Schaftform 1 beim Einschieben des Schaftteils 12 durch die proximale Öffnung 3 in die Schaftform 1 erleichtern. Die Höhe der Abstandhalter 22 ist dabei so gewählt, dass sie an der Innenwand des Innenraums 2 der Schaftform 1 anliegen, wenn das Schaftteil 12 vollständig in die Schaftform 1 eingeschoben ist. Der Anschlag hierfür kann durch die proximale Wandung 4 der Schaftform 1 und durch die distale Oberfläche 20 des Stegs 16 gebildet sein.

Am Kopfteil 14 können mehrere vorspringende Zentriereinrichtungen 24, beispielsweise in Form von vier vorstehenden Finnen angeordnet sein, die die Positionierung und Ausrichtung des Kopfteils 14 in der Kopfform 5 beim Einschieben des Kopfteils 14 durch die distale Öffnung 8 in die Kopfform 5 erleichtern. Zu dem gleichen Zweck kann das Kopfteil 14 an seiner proximalen Seite mit einem Konus 26 geformt sein, der das Einschieben in den Knochenzementteig 36 erleichtern soll. Die Ausrichtung der Finnen ist vorzugsweise so gewählt, dass sie entlang ihrer linearen Oberflächen in den Hohlraum 6 der Kopfform 5 gleiten können. Die Kopfform 5 kann eine Halsform 28 mit zylindrischem Innenraum als Teil des Hohlraums 6 aufweisen. Die Zentriereinrichtungen 24 können an der Innenwand der Halsform 28 gleiten. Die Höhe der Zentriereinrichtungen 24 ist bevorzugt dabei so gewählt, dass sie in der Innenwand des Hohlraums 6 der Halsform 28 der Kopfform 5 anliegen, wenn das Kopfteil 14 vollständig in die Kopfform 5 eingeschoben ist. Der Anschlag hierfür kann durch die distale Wandung 9 der Kopfform 5 und durch die proximale Oberfläche 18 des Stegs 16 gebildet sein. Die Halsform 28 kann mehrere vorstehende Ringe 30 in Form von umlaufenden Verdickungen aufweisen, entlang denen die Halsform 28 geschnitten und dadurch gekürzt werden kann. Die Ringe 30 können hierzu in gleichmäßigen Abständen angeordnet sein, insbesondere in gleichmäßigen ganzzahligen oder halbzahligen Zentimeter- oder Zoll-Abständen. Hierdurch kann die Länge eines Halses 44 des zu erzeugenden Spacers 38 eingestellt werden, der durch die Länge der Halsform 28 bestimmt wird.

Der Steg 16 kann mehrere Vorsprünge 32 aufweisen, die in radialer beziehungsweise transversaler Richtung von dem Metallkern 16 abstehen können. Die Vorsprünge 32 können die proximale Oberfläche 18 und/oder die distale Oberfläche 20 des Stegs 16 bilden oder Teil der proximalen Oberfläche 18 und/oder der distalen Oberfläche 20 des Stegs 16 sein. Zwischen den Zwischenräumen zwischen den Vorsprüngen 32 kann überschüssiger Knochenzementteig 36 aus der Schaftform 1 aber theoretisch auch aus der Kopfform 5 austreten, wenn der Metallkern 10 hineingeschoben wird.

An der Schaftform 1 kann ein Kragen 34 ausformt sein, der ausgehend von der proximalen Wandung 4 der Schaftform 1 den Steg 16 umgreift.

Die Schaftform 1 und die Kopfform 5 können erfindungsgemäß kostengünstig durch Spritzguss aus Kunststoff hergestellt werden oder sogar auch aus einer tiefgezogenen Kunststofffolie hergestellt werden, da sie selbst beim Einfüllen eines hochviskosen Knochenzementteigs keine sehr großen Kräfte aufnehmen müssen. Dies ist möglich, weil erfindungsgemäß der Knochenzementteig 36 in die Schaftform 1 und die Kopfform 5 als Gießform gefüllt wird, ohne dass der Metallkern 10 bereits darin angeordnet ist und deswegen umflossen werden müsste. Die Anordnung des Metallkerns 10 in der Schaftform 1 und in der Kopfform 5 im Anschluss an das Einfüllen des Knochenzementteigs 36 ist dadurch möglich, dass die Schaftform 1 und die Kopfform 5 an den Steg 16 des Metallkerns 10 angelegt werden und so passend zum Metallkern 10 und passend zueinander angeordnet und ausgerichtet werden. Dadurch müssen die Schaftform 1 und die Kopfform 5 nicht aufwendig und druckaufnehmend stabil aneinandergeschraubt beziehungsweise aneinander befestigt werden.

Nach dem Aushärten des Knochenzementteigs 36 in der aus der Schaftform 1 und der Kopfform 5 über den Metallkern 10 zusammengesetzten Gießform härtet ein darin enthaltener Knochenzementteig 36 aus (siehe Figur 6). Im Anschluss kann der so erhaltene Spacer 38 entformt werden, indem die Schaftform 1 abgezogen wird und die Kopfform 5 aufgetrennt und abgetrennt wird. Es verbleibt der Spacer 38, wie er in Figur 7 gezeigt ist, wobei der Metallkern 10 als Armierung in dem Spacer 38 eingeschlossen beziehungsweise weitgehend eingeschlossen ist. An der Oberfläche des Spacers 38 sind noch der Steg 16 beziehungsweise die Vorsprünge 32 des Stegs 16 am Übergang vom Schaft 42 zum Hals 44 des Spacers 38 zu erkennen. Ebenso können auch die äußeren Oberflächen der Abstandhalter 22 im Schaft 42 und der Zentriereinrichtungen 24 im Hals 44 des Spacers 38 zu erkennen sein.

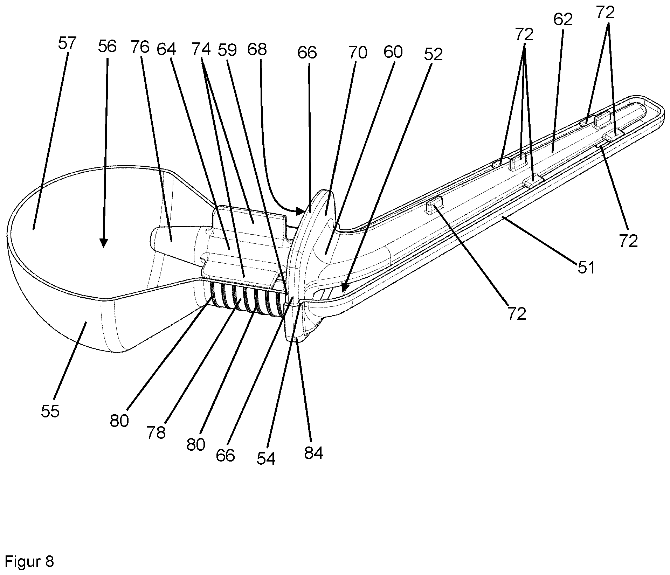

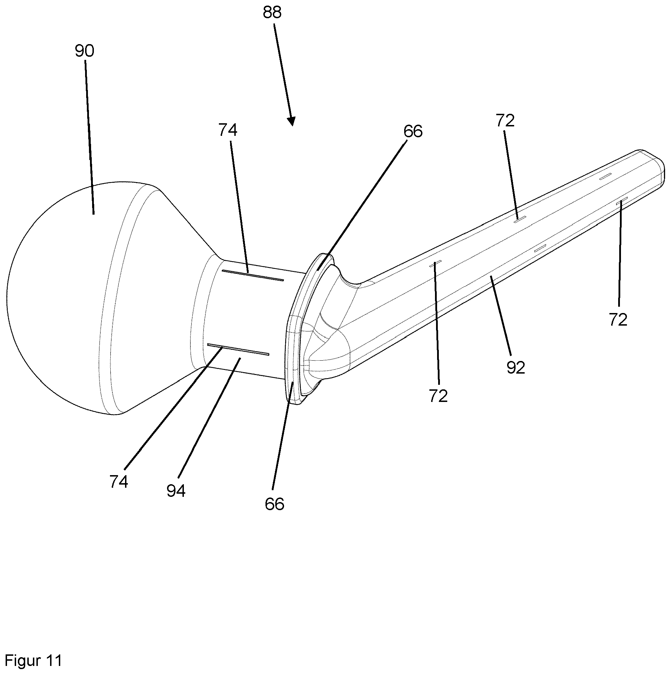

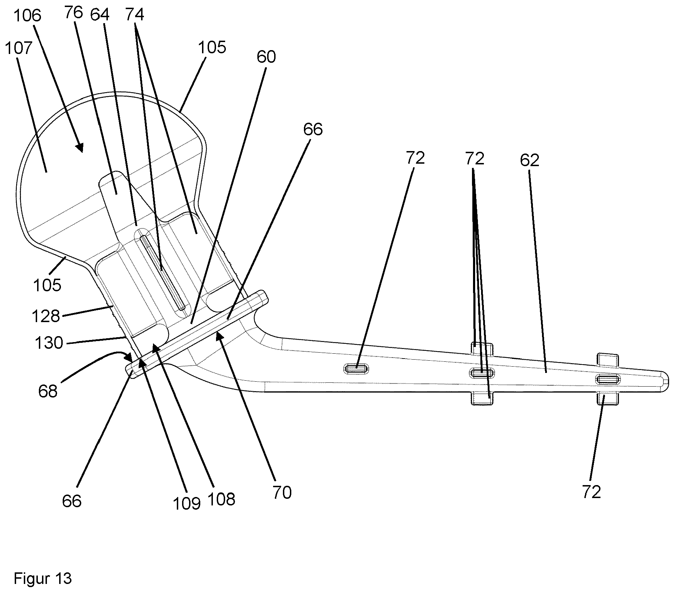

In den Figuren 8 bis 12 und 14 bis 20 sind Abbildungen eines zweiten Ausführungsbeispiels einer erfindungsgemäßen Vorrichtung zur Herstellung eines Hüftgelenkspacers, wie er in Figur 11 gezeigt ist, und Teile der Vorrichtung in verschiedenen Ansichten gezeigt. Figur 13 zeigt ein drittes Ausführungsbeispiel, das sich vom zweiten Ausführungsbeispiel lediglich durch einen abweichenden Durchmesser zum Formen des Gelenkkopfs auszeichnet, ansonsten aber identisch ist.

Die zweite erfindungsgemäße Vorrichtung und die dritte erfindungsgemäße Vorrichtung sind zur Herstellung eines Spacers 88 (siehe Figur 11) für ein Hüftgelenk geeignet und vorgesehen. Die zweite erfindungsgemäße Vorrichtung hat eine Schaftform 51 und eine Kopfform 55 und einen Metallkern 60. Die Schaftform 51 und die Kopfform 55 sind vorzugsweise jeweils einteilig aufgebaut. Die Schaftform 51 und die Kopfform 55 können gemeinsam eine zweiteilige Gießform zum Formen eines darin eingefüllten Knochenzementteigs 86 (siehe Figuren 14 bis 20) bilden. Hierzu härtet der Knochenzementteig 86 in der Gießform aus. In den Figuren 8, 10, 12 und 14 bis 20 sind die Schaftform 51 und die Kopfform 55 geschnitten dargestellt, so dass der innere Aufbau der Vorrichtung erkennbar ist. Der Metallkern 60 ist immer ungeschnitten dargestellt.

Die Schaftform 51 weist einen Innenraum 52 auf, in dem der Knochenzementteig 86 zu einem Schaft 92 des Spacers 88 formbar ist. Der Innenraum 52 ist über eine proximale Öffnung 53 zugänglich, so dass der Knochenzementteig 86 durch die proximale Öffnung 53 der Schaftform 51 in den Innenraum 52 einfüllbar ist und der Metallkern 60 teilweise durch die proximale Öffnung 53 in den Innenraum 52 der Schaftform 51 einschiebbar ist. Um die proximale Öffnung 53 herum weist die Schaftform 51 eine proximale Wandung 54 auf, die die proximale Öffnung 53 begrenzt und umschließt. Die Kopfform 55 weist einen Hohlraum 56 auf, in dem der Knochenzementteig 86 zu einem Kopf 90 des Spacers 88 formbar ist. Der Hohlraum 56 weist an einer proximalen Seite des Hohlraums 56 eine kugelflächenförmige Innenfläche 57 auf, die zum Ausformen der eigentlichen Gelenkfläche des Spacers 88 dient. Dementsprechend formt die kugelflächenförmige Innenfläche 57 des Hohlraums 56 die Hüftgelenkoberfläche des Spacers 88, die in einer Hüftpfanne gleiten soll. Der Hohlraum 56 ist über eine distale Öffnung 58 zugänglich, so dass der Knochenzementteig 86 durch die distale Öffnung 58 der Kopfform 55 in den Hohlraum 56 einfüllbar ist und der Metallkern 60 teilweise durch die distale Öffnung 58 in den Hohlraum 56 der Kopfform 55 einschiebbar ist. Um die distale Öffnung 58 herum weist die Kopfform 55 eine distale Wandung 59 auf, die die distale Öffnung 58 begrenzt und umschließt.

Der Metallkern 60 kann aus einem biokompatiblen Metall, wie beispielsweise Edelstahl oder Titan bestehen. Der Metallkern 60 dient der Armierung und damit der mechanischen Stabilisierung des Spacers 88. Der Metallkern 60 hat ein Schaftteil 62, ein Kopfteil 64 und einen Steg 66, der zwischen dem Schaftteil 62 und dem Kopfteil 64 angeordnet ist. Vorzugsweise ist der Metallkern 60 einteilig. Das Schaftteil 62 und das Kopfteil 64 können über den Steg 66 miteinander verbunden sein. Der Steg 66 kann in radialer Richtung von den Achsen des Kopfteils 64 und des Schaftteils 62 abstehen. Der vorspringende Steg 66 weist eine proximale Oberfläche 68 und eine gegenüberliegende distale Oberfläche 70 auf.

Die proximale Wandung 54 der Schaftform 51 ist passend zu der distalen Oberfläche 70 des Stegs 66 geformt, so dass die Schaftform 51 zumindest bereichsweise flächenbündig and die distale Oberfläche 70 des Stegs 66 anlegbar ist. Dies erlaubt eine eindeutige Positionierung der Schaftform 51 an den Steg 66 des Metallkerns 60. In gleicher Weise ist die distale Wandung 59 der Kopfform 55 passend zu der proximalen Oberfläche 68 des Stegs 66 geformt, so dass die Kopfform 55 zumindest bereichsweise flächenbündig an die proximale Oberfläche 68 des Stegs 66 anlegbar ist. Dies erlaubt eine eindeutige Positionierung der Kopfform 55 an den Steg 66 des Metallkerns 60.

Am Schaftteil 62 können mehrere vorspringende Abstandhalter 72 mit länglicher Form angeordnet sein, die die Positionierung und Ausrichtung des Schaftteils 62 in der Schaftform 51 beim Einschieben des Schaftteils 62 durch die proximale Öffnung 53 in die Schaftform 51 erleichtern. Die Höhe der Abstandhalter 72 ist dabei so gewählt, dass sie an der Innenwand des Innenraums 52 der Schaftform 51 anliegen, wenn das Schaftteil 62 vollständig in die Schaftform 51 eingeschoben ist. Der Anschlag hierfür kann durch die proximale Wandung 54 der Schaftform 51 und durch die distale Oberfläche 70 des Stegs 66 gebildet sein.

Am Kopfteil 64 können mehrere vorspringende Zentriereinrichtungen 74, beispielsweise in Form von vier vorstehenden Finnen angeordnet sein, die die Positionierung und Ausrichtung des Kopfteils 64 in der Kopfform 55 beim Einschieben des Kopfteils 64 durch die distale Öffnung 58 in die Kopfform 55 erleichtern. Zu dem gleichen Zweck kann das Kopfteil 64 an seiner proximalen Seite mit einem Konus 76 geformt sein, der das Einschieben in den Knochenzementteig 86 erleichtern soll. Die Ausrichtung der Finnen ist vorzugsweise so gewählt, dass sie entlang ihrer linearen Oberflächen in den Hohlraum 56 der Kopfform 55 gleiten können. Die Kopfform 55 kann eine Halsform 78 mit zylindrischem Innenraum als Teil des Hohlraums 56 aufweisen. Die Zentriereinrichtungen 74 können an der Innenwand der Halsform 78 gleiten. Die Höhe der Zentriereinrichtungen 74 ist bevorzugt dabei so gewählt, dass sie in der Innenwand des Hohlraums 56 der Halsform 78 der Kopfform 55 anliegen, wenn das Kopfteil 64 vollständig in die Kopfform 55 eingeschoben ist. Der Anschlag hierfür kann durch die distale Wandung 59 der Kopfform 55 und durch die proximale Oberfläche 68 des Stegs 66 gebildet sein.

Die Halsform 78 kann mehrere vorstehende Ringe 80 in Form von umlaufenden Verdickungen aufweisen, entlang denen die Halsform 78 geschnitten und dadurch gekürzt werden kann. Die Ringe 80 können hierzu in gleichmäßigen Abständen angeordnet sein, insbesondere in gleichmäßigen ganzzahligen oder halbzahligen Zentimeter- oder Zoll-Abständen. Hierdurch kann die Länge eines Halses 94 des zu erzeugenden Spacers 88 eingestellt werden, der durch die Länge der Halsform 78 bestimmt wird.

An der Schaftform 51 kann ein Kragen 84 ausformt sein, der ausgehend von der proximalen Wandung 54 der Schaftform 51 den Steg 66 umgreift.

Die Schaftform 51 und die Kopfform 55 können erfindungsgemäß kostengünstig durch Spritzguss aus Kunststoff hergestellt werden oder sogar auch aus einer tiefgezogenen Kunststofffolie hergestellt werden, da sie selbst beim Einfüllen eines hochviskosen Knochenzementteigs keine sehr großen Kräfte aufnehmen müssen. Dies ist möglich, weil erfindungsgemäß der Knochenzementteig 86 in die Schaftform 51 und die Kopfform 55 als Gießform gefüllt wird, ohne dass der Metallkern 60 bereits darin angeordnet ist und deswegen umflossen werden müsste. Die Anordnung des Metallkerns 60 in der Schaftform 51 und in der Kopfform 55 im Anschluss an das Einfüllen des Knochenzementteigs 86 ist dadurch möglich, dass die Schaftform 51 und die Kopfform 55 an den Steg 66 des Metallkerns 60 angelegt werden und so passend zum Metallkern 60 und passend zueinander angeordnet und ausgerichtet werden. Dadurch müssen die Schaftform 51 und die Kopfform 55 nicht aufwendig und druckaufnehmend stabil aneinandergeschraubt beziehungsweise aneinander befestigt werden.

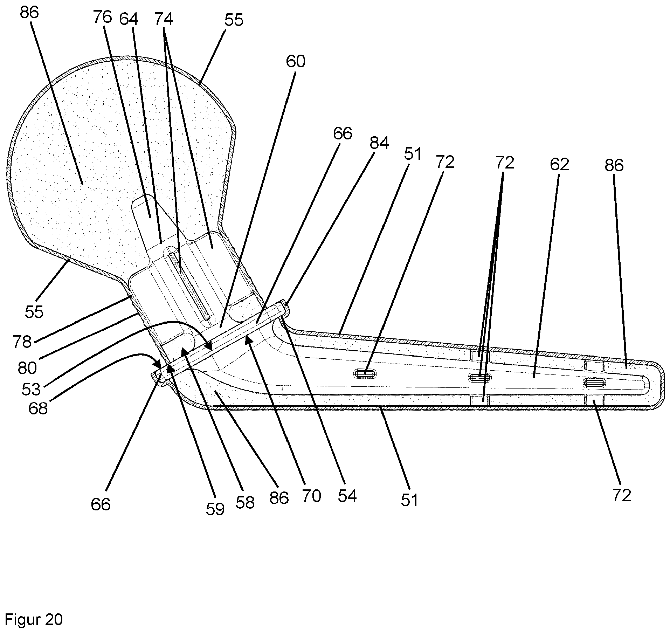

Nach dem Aushärten des Knochenzementteigs 86 in der aus der Schaftform 51 und der Kopfform 55 über den Metallkern 60 zusammengesetzten Gießform härtet ein darin enthaltener Knochenzementteig 86 aus (siehe Figur 20). Im Anschluss kann der so erhaltene Spacer 38 entformt werden, indem die Schaftform 51 abgezogen wird und die Kopfform 55 aufgetrennt und abgetrennt wird. Es verbleibt der Spacer 88, wie er in Figur 11 gezeigt ist, wobei der Metallkern 60 als Armierung in dem Spacer 88 eingeschlossen beziehungsweise weitgehend eingeschlossen ist. An der Oberfläche des Spacers 88 sind noch der Steg 66 am Übergang vom Schaft 92 zum Hals 94 des Spacers 88 zu erkennen. Ebenso können auch die äußeren Oberflächen der Abstandhalter 72 im Schaft 92 und der Zentriereinrichtungen 74 im Hals 94 des Spacers 88 zu erkennen sein.

Der Knochenzementteig 86 kann vor dem Einfüllen in die Schaftform 51 oder in die Kopfform 55 aus einer Monomerflüssigkeit und einem Zementpulver (nicht gezeigt) gemischt worden sein, wobei dem Knochenzementteig 86 vorzugsweise auch zumindest ein Antibiotikum und/oder zumindest ein Antimykotikum beigemischt werden können. Der Knochenzementteig 86 kann über einen Knochenzementapplikator 132 in die Schaftform 51 und in die Kopfform 55 gefüllt werden (siehe Figuren 14 und 17). Dabei werden der Innenraum 52 der Schaftform 51 von der distalen Seite aus und der Hohlraum 56 der Kopfform 55 von der proximalen Seite aus gefüllt, um Lufteinschlüsse zu verhindern. Zur Vermeidung von Lufteinschlüssen können auch Entgasungsöffnungen (nicht gezeigt) in den Wandungen der Schaftform 51 und der Kopfform 52 vorgesehen sein.

Nachdem der Knochenzementteig 86 im Überschuss in die Schaftform 51 und die Kopfform 55 gefüllt wurde, wird das Schaftteil 62 des Metallkern 60 in die Schaftform 51 eingeschoben und das Kopfteil 64 des Metallkerns 60 in die Kopfform 55 eingeschoben.

Der Steg 66 bildet für beides den Anschlag. Austretender überschüssiger Knochenzementteig 86 kann entfernt werden. Der Knochenzementteig 86 härtet in diesem Zustand mit dem eingeschlossenen Metallkern 60 darin aus (siehe Figur 20).

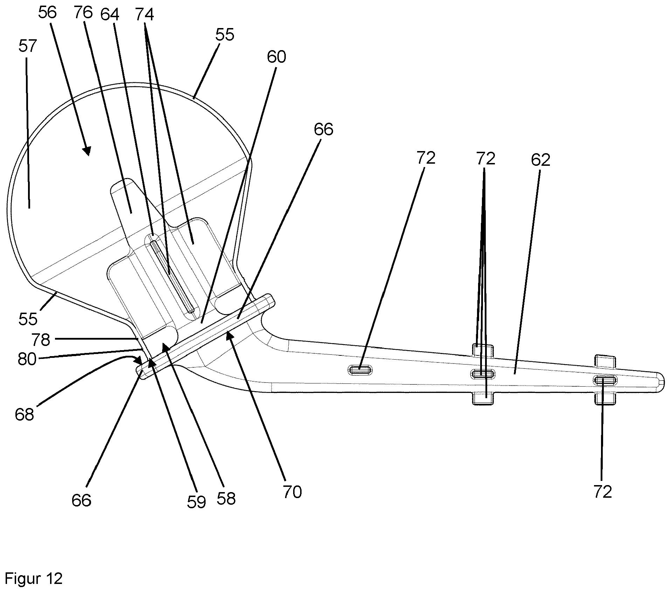

Zur Anpassung des Spacers 88 an die anatomischen Gegebenheiten kann der Hals 78 entlang der Ringe 80 gekürzt werden, so wie das in Figur 12 gezeigt ist.

Das in Figur 13 gezeigte dritte Ausführungsbeispiel unterscheidet sich von dem zweiten Ausführungsbeispiel lediglich dadurch, dass dieses eine andere Kopfform 105 mit einem anderen Radius einer kugelflächenförmigen Innenfläche 107 aufweist. Durch die andere Form der Kopfform 105 ist auch eine anderer Hohlraum 106 in der Kopfform 105 enthalten. Die Kopfform 105 weist analog zur Kopfform 55 eine distale Öffnung 108 und eine umlaufende distale Wandung 109.

Eine Halsform 128 mit umlaufenden Ringen 130 kann ebenfalls vorgesehen sein. Alle anderen Teile des dritten Ausführungsbeispiels gleichen dem zweiten Ausführungsbeispiel. Die Kopfform 105 kann zusammen mit der Kopfform 55 sowie mit dem Metallkern 60 und der Schaftform 51 und wenn gewünscht auch mit dem Knochenzementapplikator 132 als ein erfindungsgemäßes Set vorliegen.

Der Knochenzementapplikator 132 kann eine Kartusche 134 zum Mischen des Knochenzementteigs 86 aufweisen, die an der Vorderseite durch einen Kartuschenkopf 136 bis auf eine Öffnung verschlossen sein kann. Zusätzlich kann ein Vakuumanschluss 138 an dem Kartuschenkopf 136 angeordnet sein, so dass der Knochenzementteig 86 im Inneren der Kartusche 134 unter Vakuum gemischt werden kann. Der fertig gemischte Knochenzementteig m86 kann durch die Öffnung und durch ein Austragsrohr 140 in die Schaftform 51 und in die Kopfform 55 gedrückt werden (siehe Figuren 14 und 17). In dem Austragsrohr 140 kann ein statischer Mischer (nicht gezeigt) angeordnet sein, mit die Ausgangskomponenten des Knochenzementteigs 86 durchmischt werden können. Das Austragsrohr 140 kann mit einer Überwurfmutter 142 an dem Kartuschenkopf 136 befestigt sein.

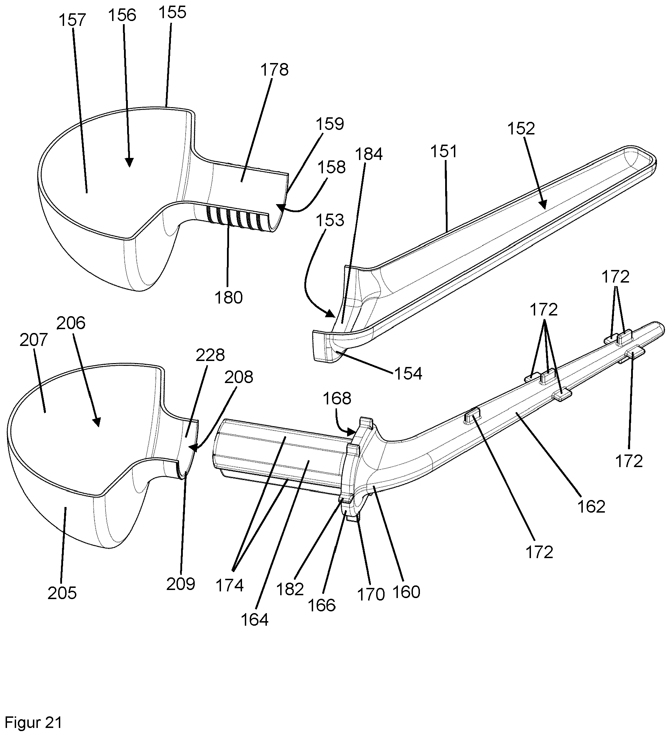

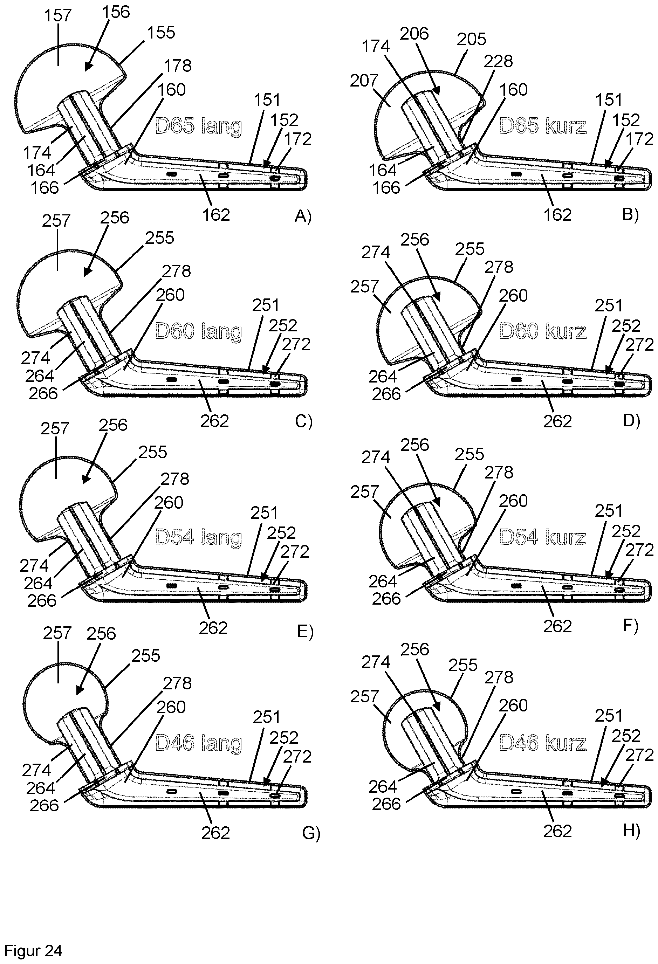

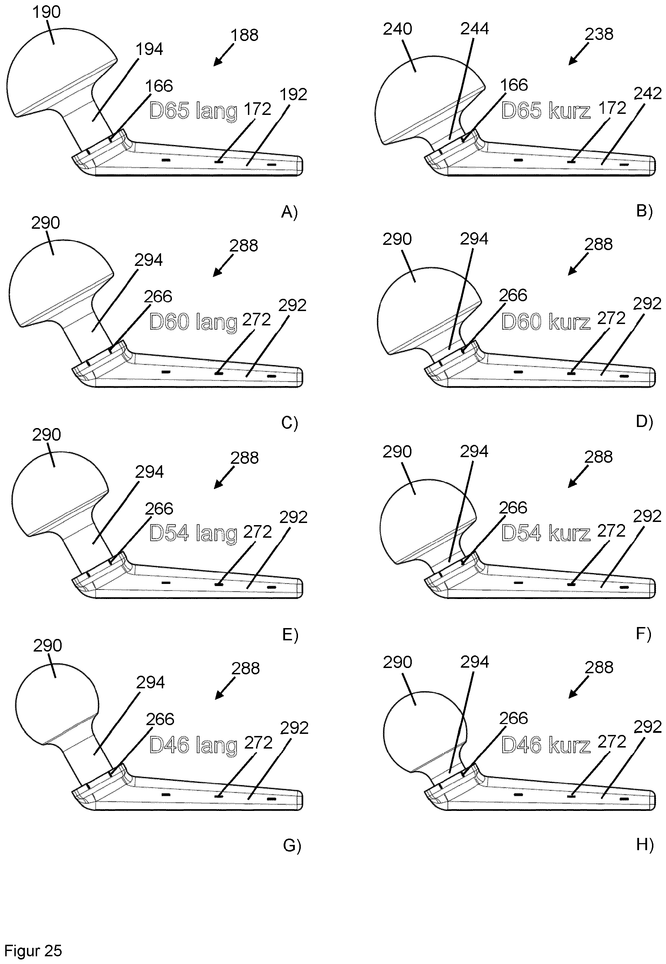

In den Figuren 21 bis 23 sowie 24 A), 24 B), 25 A) und 25 B) sind Abbildungen eines erfindungsgemäßen Sets und eines vierten Ausführungsbeispiels einer erfindungsgemäßen Vorrichtung zur Herstellung eines Hüftgelenkspacers, wie er in den Figuren 25 A) und 25 B) gezeigt ist, und Teile der Vorrichtung in verschiedenen Ansichten gezeigt. Analog der Kombination der unterschiedlichen Kopfformen des zweiten und dritten Ausführungsbeispiels hat das Set zwei unterschiedlich geformte Kopfformen 155, 205. Die Figuren 24 C) bis 24 H) und 25 C) bis 25 H) zeigen Alternativen mit gekürzten und ungekürzten Kopfformen 255 mit kugelflächenförmigen Innenflächen 257 mit unterschiedlichen Radien.

Das Set beziehungsweise die vierte erfindungsgemäße Vorrichtung ist zur Herstellung zweier unterschiedlich geformter Spacers 188, 238 (siehe Figuren 25 A) und 25 B)) für ein Hüftgelenk geeignet und vorgesehen. Die Vorrichtung hat eine Schaftform 151 und die zwei Kopfformen 155, 205 sowie einen Metallkern 160. Die Schaftform 151 und die Kopfformen 155, 205 sind vorzugsweise jeweils einteilig aufgebaut. Die Schaftform 151 und die Kopfformen 155, 205 können gemeinsam zwei zweiteilige Gießformen zum Formen eines darin eingefüllten Knochenzementteigs (nicht gezeigt aber analog Figur 6 und 20) bilden. Hierzu härtet der Knochenzementteig in der Gießform aus. In den Figuren 21, 23 und 24 sind die Schaftform 151 und die Kopfformen 155, 205 geschnitten dargestellt, so dass der innere Aufbau der Vorrichtung erkennbar ist. Der Metallkern 160 ist immer ungeschnitten dargestellt. Die Schaftform 151 weist einen Innenraum 152 auf, in dem der Knochenzementteig zu einem Schaft 192, 242 des Spacers 188, 238 formbar ist. Der Innenraum 152 ist über eine proximale Öffnung 153 zugänglich, so dass der Knochenzementteig durch die proximale Öffnung 153 der Schaftform 151 in den Innenraum 152 einfüllbar ist und der Metallkern 160 teilweise durch die proximale Öffnung 153 in den Innenraum 152 der Schaftform 151 einschiebbar ist. Um die proximale Öffnung 153 herum weist die Schaftform 151 eine proximale Wandung 154 auf, die die proximale Öffnung 153 begrenzt und umschließt.

Die Kopfformen 155, 205 weisen einen Hohlraum 156, 206 auf, in dem der Knochenzementteig zu einem Kopf 190 des Spacers 188 formbar ist. Jeder der Hohlräume 156, 206 weist an einer proximalen Seite des Hohlraums 156, 206 eine kugelflächenförmige Innenfläche 157, 207 auf, die zum Ausformen der eigentlichen Gelenkfläche des Spacers 188, 238 dient. Dementsprechend formen die kugelflächenförmigen Innenflächen 157, 207 der Hohlräume 156, 206 die Hüftgelenkoberflächen der Spacers 188, 238, die in einer Hüftpfanne gleiten sollen. Die Hohlräume 156, 206 sind über eine distale Öffnung 158, 208 zugänglich, so dass der Knochenzementteig durch die distale Öffnung 158, 208 der Kopfformen 155, 205 in die Hohlräume 156, 206 einfüllbar ist und der Metallkern 160 teilweise durch die distale Öffnungen 158, 208 in die Hohlräume 156, 206 der Kopfformen 155, 205 einschiebbar ist. Um die distale Öffnung 158, 208 herum weisen die Kopfformen 155, 205 je eine distale Wandung 159, 209 auf, die die jeweilige distale Öffnung 158, 208 begrenzt und umschließt.

Der Knochenzementteig kann vor dem Einfüllen aus einer Monomerflüssigkeit und einem Zementpulver (nicht gezeigt) gemischt worden sein, wobei dem Knochenzementteig vorzugsweise auch zumindest ein Antibiotikum und/oder zumindest ein Antimykotikum beigemischt werden können. Der Knochenzementteig kann über einen Knochenzementapplikator 132, wie er in den Figuren 14 und 17 zum zweiten Ausführungsbeispiel gezeigt ist, in die Schaftform 151 und in die Kopfformen 155, 205 gefüllt werden.

Der Metallkern 160 kann aus einem biokompatiblen Metall, wie beispielsweise Edelstahl oder Titan bestehen. Der Metallkern 160 dient der Armierung und damit der mechanischen Stabilisierung des Spacers 188, 238. Der Metallkern 160 hat ein Schaftteil 162, ein Kopfteil 164 und einen Steg 166, der zwischen dem Schaftteil 162 und dem Kopfteil 164 angeordnet ist. Vorzugsweise ist der Metallkern 160 einteilig. Das Schaftteil 162 und das Kopfteil 164 können über den Steg 166 miteinander verbunden sein. Der Steg 166 kann in radialer Richtung von den Achsen des Kopfteils 164 und des Schaftteils 162 abstehen. Der vorspringende Steg 166 weist eine proximale Oberfläche 168 und eine gegenüberliegende distale Oberfläche 170 auf.

Die proximale Wandung 154 der Schaftform 151 ist passend zu der distalen Oberfläche 170 des Stegs 166 geformt, so dass die Schaftform 151 zumindest bereichsweise flächenbündig and die distale Oberfläche 170 des Stegs 166 anlegbar ist. Dies erlaubt eine eindeutige Positionierung der Schaftform 151 an den Steg 166 des Metallkerns 160. In gleicher Weise ist die distale Wandung 159, 209 der Kopfformen 155, 205 passend zu der proximalen Oberfläche 168 des Stegs 166 geformt, so dass die Kopfformen 155, 205 zumindest bereichsweise flächenbündig an die proximale Oberfläche 168 des Stegs 166 anlegbar ist. Dies erlaubt eine eindeutige Positionierung der Kopfformen 155, 205 an den Steg 166 des Metallkerns 160.

Am Schaftteil 162 können mehrere vorspringende Abstandhalter 172 mit länglicher Form angeordnet sein, die die Positionierung und Ausrichtung des Schaftteils 172 in der Schaftform 151 beim Einschieben des Schaftteils 162 durch die proximale Öffnung 153 in die Schaftform 151 erleichtern. Die Höhe der Abstandhalter 172 ist dabei so gewählt, dass sie an der Innenwand des Innenraums 152 der Schaftform 151 anliegen, wenn das Schaftteil 162 vollständig in die Schaftform 151 eingeschoben ist. Der Anschlag hierfür kann durch die proximale Wandung 154 der Schaftform 151 und durch die distale Oberfläche 170 des Stegs 166 gebildet sein. Am Kopfteil 164 können mehrere vorspringende Zentriereinrichtungen 174, beispielsweise in Form von vier vorstehenden Finnen angeordnet sein, die die Positionierung und Ausrichtung des Kopfteils 164 in die Kopfformen 155, 205 beim Einschieben des Kopfteils 164 durch die jeweilige distale Öffnung 158, 208 erleichtern. Die Ausrichtung der Finnen ist vorzugsweise so gewählt, dass sie entlang ihrer linearen Oberflächen in die Hohlräume 156, 206 der Kopfformen 155, 205 gleiten können. Die Kopfformen 155, 205 können unterschiedlich lange Halsformen 178, 228 mit zylindrischem Innenraum als Teil der Hohlräume 156, 206 aufweisen. Die Zentriereinrichtungen 174 können an den zylindrischen Innenwänden der Halsformen 178, 228 gleiten. Die Höhe der Zentriereinrichtungen 174 ist bevorzugt dabei so gewählt, dass sie in den zylindrischen Innenwänden des Hohlraums 156, 206 der Halsformen 178, 228 anliegen, wenn das Kopfteil 164 vollständig in die Kopfformen 155, 205 eingeschoben ist. Der Anschlag hierfür kann durch die distalen Wandungen 159, 209 der Kopfformen 155, 205 und durch die proximale Oberfläche 168 des Stegs 166 gebildet sein.

Die Halsform 178 kann mehrere vorstehende Ringe 180 in Form von umlaufenden Verdickungen aufweisen, entlang denen die Halsform 178 geschnitten und dadurch gekürzt werden kann. Die Halsform 228 kann als eine maximal gekürzte Halsform 178 aufgefasst werden. Die Ringe 180 können in gleichmäßigen Abständen angeordnet sein, insbesondere in gleichmäßigen ganzzahligen oder halbzahligen Zentimeter- oder Zoll-Abständen. Hierdurch kann die Länge eines Halses 194, 244 des zu erzeugenden Spacers 188, 238 eingestellt werden, der durch die Länge der jeweiligen Halsform 178, 228 bestimmt wird.

Der Steg 166 kann mehrere Vorsprünge 182 aufweisen, die in radialer beziehungsweise transversaler Richtung von dem Metallkern 166 abstehen können. Die Vorsprünge 182 können die proximale Oberfläche 168 und/oder die distale Oberfläche 170 des Stegs 166 bilden oder Teil der proximalen Oberfläche 168 und/oder der distalen Oberfläche 170 des Stegs 166 sein. Zwischen den Zwischenräumen zwischen den Vorsprüngen 182 kann überschüssiger Knochenzementteig aus der Schaftform 151 aber theoretisch auch aus den Kopfformen 155, 205 austreten, wenn der Metallkern 160 hineingeschoben wird.

An der Schaftform 151 kann ein Kragen 184 ausformt sein, der ausgehend von der proximalen Wandung 154 der Schaftform 151 den Steg 166 umgreift.

Die Schaftform 151 und die Kopfformen 155, 205 können erfindungsgemäß kostengünstig durch Spritzguss aus Kunststoff hergestellt werden oder sogar auch aus einer tiefgezogenen Kunststofffolie hergestellt werden, da sie selbst beim Einfüllen eines hochviskosen Knochenzementteigs keine sehr großen Kräfte aufnehmen müssen. Dies ist möglich, weil erfindungsgemäß der Knochenzementteig in die Schaftform 151 und die Kopfformen 155, 205 als Gießform gefüllt wird, ohne dass der Metallkern 160 bereits darin angeordnet ist und deswegen umflossen werden müsste. Die Anordnung des Metallkerns 160 in der Schaftform 151 und in eine der Kopfformen 155, 205 im Anschluss an das Einfüllen des Knochenzementteigs ist dadurch möglich, dass die Schaftform 151 und die Kopfformen 155, 205 an den Steg 166 des Metallkerns 160 angelegt werden und so passend zum Metallkern 160 und passend zueinander angeordnet und ausgerichtet werden. Dadurch müssen die Schaftform 151 und die Kopfformen 155, 205 nicht aufwendig und druckaufnehmend stabil aneinandergeschraubt beziehungsweise aneinander befestigt werden.

Nach dem Aushärten des Knochenzementteigs in der aus der Schaftform 151 und einer der Kopfformen 155, 205 über den Metallkern 160 zusammengesetzten Gießform härtet ein darin enthaltener Knochenzementteig aus.

Im Anschluss kann der so erhaltene Spacer 188, 238 entformt werden, indem die Schaftform 151 abgezogen wird und die benutzte Kopfform 155, 205 aufgetrennt und abgetrennt wird. Es verbleibt der Spacer 188, 238, wie er in Figur 25 gezeigt ist, wobei der Metallkern 160 als Armierung in dem Spacer 188, 238 eingeschlossen beziehungsweise weitgehend eingeschlossen ist. An der Oberfläche des Spacers 188, 238 sind noch der Steg 166 beziehungsweise die Vorsprünge 182 des Stegs 166 am Übergang vom Schaft 192, 242 zum Hals 294, 244 des Spacers 188, 238 zu erkennen. Ebenso können auch die äußeren Oberflächen der Abstandhalter 172 im Schaft 192, 242 und der Zentriereinrichtungen 174 im Hals 194, 244 des Spacers 188, 238 zu erkennen sein.

Die weiteren Varianten, die in den Figuren 24 C) bis 24 H) gezeigt sind und mit denen Spacer 288, die in den Figuren 25 C) bis 25 H) für ein Hüftgelenk hergestellt werden können, gleichen bis auf geringere Durchmesser der kugelflächenförmigen Innenflächen 257 denen des Sets nach den Figuren 21 bis 23 sowie 24 A), 24 B), 25 A) und 25 B). Der Einfachheit halber wurden bei all diesen Ausführungsbeispielen auch bei unterschiedlicher Form die gleichen Bezugszeichen verwendet.

Die Vorrichtungen haben eine Schaftform 251 und eine Kopfform 255 und einen Metallkern 260. Die Schaftform 251 und die Kopfform 255 sind vorzugsweise jeweils einteilig aufgebaut. Die Schaftform 251 und die Kopfform 255 können gemeinsam eine zweiteilige Gießform zum Formen eines darin eingefüllten Knochenzementteigs (nicht gezeigt) bilden.

Die Schaftform 251 weist einen Innenraum 252 auf, in dem der Knochenzementteig zu einem Schaft 292 des Spacers 288 formbar ist. Der Innenraum 252 ist über eine proximale Öffnung zugänglich, so dass der Knochenzementteig durch die proximale Öffnung der Schaftform 251 in den Innenraum 252 einfüllbar ist und der Metallkern 260 teilweise durch die proximale Öffnung in den Innenraum 252 der Schaftform 251 einschiebbar ist. Um die proximale Öffnung herum weist die Schaftform 251 eine proximale Wandung auf, die die proximale Öffnung begrenzt und umschließt.

Die Kopfform 255 weist einen Hohlraum 256 auf, in dem der Knochenzementteig zu einem Kopf 290 des Spacers 288 formbar ist. Der Hohlraum 256 weist an einer proximalen Seite des Hohlraums 256 eine kugelflächenförmige Innenfläche 257 mit unterschiedlichen Durchmessern auf, die zum Ausformen der eigentlichen Gelenkfläche des Spacers 288 dient. Dementsprechend formt die kugelflächenförmige Innenfläche 257 des Hohlraums 256 die Hüftgelenkoberfläche des Spacers 288, die in einer Hüftpfanne gleiten soll. Der Hohlraum 256 ist über eine distale Öffnung zugänglich, so dass der Knochenzementteig durch die distale Öffnung der Kopfform 255 in den Hohlraum 256 einfüllbar ist und der Metallkern 260 teilweise durch die distale Öffnung in den Hohlraum 256 der Kopfform 255 einschiebbar ist. Um die distale Öffnung herum weist die Kopfform 255 eine distale Wandung auf, die die distale Öffnung begrenzt und umschließt.

Der Metallkern 160 kann aus einem biokompatiblen Metall, wie beispielsweise Edelstahl oder Titan bestehen. Der Metallkern 160 dient der Armierung und damit der mechanischen Stabilisierung des Spacers 288. Der Metallkern 260 hat ein Schaftteil 262, ein Kopfteil 264 und einen Steg 266, der zwischen dem Schaftteil 262 und dem Kopfteil 264 angeordnet ist. Vorzugsweise ist der Metallkern 260 einteilig. Das Schaftteil 262 und das Kopfteil 264 können über den Steg 266 miteinander verbunden sein. Der Steg 266 kann in radialer Richtung von den Achsen des Kopfteils 264 und des Schaftteils 262 abstehen. Der vorspringende Steg 266 weist eine proximale Oberfläche und eine gegenüberliegende distale Oberfläche auf.

Am Schaftteil 262 können mehrere vorspringende Abstandhalter 272 mit länglicher Form angeordnet sein, die die Positionierung und Ausrichtung des Schaftteils 262 in der Schaftform 251 beim Einschieben des Schaftteils 262 durch die proximale Öffnung in die Schaftform 251 erleichtern. Die Höhe der Abstandhalter 272 ist dabei so gewählt, dass sie an der Innenwand des Innenraums 252 der Schaftform 251 anliegen, wenn das Schaftteil 262 vollständig in die Schaftform 251 eingeschoben ist.

Am Kopfteil 264 können mehrere vorspringende Zentriereinrichtungen 274, beispielsweise in Form von vier vorstehenden Finnen angeordnet sein, die die Positionierung und Ausrichtung des Kopfteils 264 in der Kopfform 255 beim Einschieben des Kopfteils 264 durch die distale Öffnung in die Kopfform 255 erleichtern. Die Ausrichtung der Finnen ist vorzugsweise so gewählt, dass sie entlang ihrer linearen Oberflächen in den Hohlraum 256 der Kopfform 255 gleiten können. Die Kopfform 255 kann eine Halsform 278 mit zylindrischem Innenraum als Teil des Hohlraums 256 aufweisen. Die Zentriereinrichtungen 274 können an der Innenwand der Halsform 278 gleiten. Die Höhe der Zentriereinrichtungen 274 ist bevorzugt dabei so gewählt, dass sie in der Innenwand des Hohlraums 256 der Halsform 278 der Kopfform 255 anliegen, wenn das Kopfteil 264 vollständig in die Kopfform 255 eingeschoben ist.

Die Schaftform 251 und die Kopfform 255 können erfindungsgemäß kostengünstig durch Spritzguss aus Kunststoff hergestellt werden oder sogar auch aus einer tiefgezogenen Kunststofffolie hergestellt werden, da sie selbst beim Einfüllen eines hochviskosen Knochenzementteigs keine sehr großen Kräfte aufnehmen müssen. Dies ist möglich, weil erfindungsgemäß der Knochenzementteig in die Schaftform 251 und die Kopfform 255 als Gießform gefüllt wird, ohne dass der Metallkern 260 bereits darin angeordnet ist und deswegen umflossen werden müsste. Die Anordnung des Metallkerns 260 in der Schaftform 251 und in der Kopfform 255 im Anschluss an das Einfüllen des Knochenzementteigs ist dadurch möglich, dass die Schaftform 251 und die Kopfform 255 an den Steg 266 des Metallkerns 260 angelegt werden und so passend zum Metallkern 260 und passend zueinander angeordnet und ausgerichtet werden. Dadurch müssen die Schaftform 251 und die Kopfform 255 nicht aufwendig und druckaufnehmend stabil aneinandergeschraubt beziehungsweise aneinander befestigt werden.

Nach dem Aushärten des Knochenzementteigs in der aus der Schaftform 251 und der Kopfform 255 über den Metallkern 260 zusammengesetzten Gießform härtet ein darin enthaltener Knochenzementteig aus. Im Anschluss kann der so erhaltene Spacer 288 entformt werden, indem die Schaftform 251 abgezogen wird und die Kopfform 255 aufgetrennt und abgetrennt wird. Es verbleibt der Spacer 288, wie er in Figur 25 gezeigt ist, wobei der Metallkern 260 als Armierung in dem Spacer 288 eingeschlossen beziehungsweise weitgehend eingeschlossen ist. An der Oberfläche des Spacers 288 sind noch der Steg 266 am Übergang vom Schaft 292 zum Hals 294 des Spacers 288 zu erkennen. Ebenso können auch die äußeren Oberflächen der Abstandhalter 272 im Schaft 292 und der Zentriereinrichtungen 274 im Hals 294 des Spacers 288 zu erkennen sein.

### Bezugszeichenliste

- 1, 51, 151, 251: Schaftform
- 2, 52, 152, 252: Innenraum
- 3, 53, 153: Proximale Öffnung der Schaftform
- 4, 54, 154: Proximale Wandung der Schaftform
- 5, 55, 105, 155, 205, 255: Kopfform
- 6, 56, 106, 156, 206, 256: Hohlraum
- 7, 57, 107, 157, 207, 257: Kugelflächenförmige Innenfläche
- 8, 58, 108, 158: distale Öffnung der Kopfform
- 9, 59, 109, 159: distale Wandung der Kopfform
- 10, 60, 160, 260: Metallkern
- 12, 62, 162, 262: Schaftteil
- 14, 64, 164, 264: Kopfteil
- 16, 66, 166, 266: Steg
- 18, 68, 168: Proximale Oberfläche des Stegs
- 20, 70, 170: Distale Oberfläche des Stegs
- 22, 72, 172, 272: Abstandhalter
- 24, 74, 174, 274: Zentriereinrichtung
- 26, 76: Konus
- 28, 78, 128, 178, 228, 278: Halsform
- 30, 80, 130, 180: Ring
- 32, 182: Vorsprung
- 34, 84, 154: Kragen
- 36, 86: Knochenzementteig
- 38, 88, 188, 238, 288: Spacer
- 40, 90, 190, 240, 290: Kopf des Spacers
- 42, 92, 192, 242, 292: Schaft des Spacers
- 44, 94, 194, 244, 294: Hals des Spacers
- 132: Knochenzementapplikator
- 134: Kartusche
- 136: Kartuschenkopf
- 138: Vakuumanschluss
- 140: Austragsrohr
- 142: Überwurfmutter

## Patentansprüche

1. Vorrichtung zum Herstellen eines Spacers (38, 88, 188, 238, 288) durch Aushärten von Knochenzementteig (36, 86), wobei der Spacer (38, 88, 188, 238, 288) dazu vorgesehen ist, im medizinischen Bereich ein Gelenk oder einen Teil eines Gelenks umfassend eine artikulierende Oberfläche eines Kopfs des Gelenks temporär zu ersetzen, insbesondere eines Hüftgelenks oder eines Schultergelenks temporär zu ersetzen, die Vorrichtung aufweisend
eine Schaftform (1, 51, 151, 251) zum Formen eines Schafts (42, 92, 192, 242, 292) des Spacers (38, 88, 188, 238, 288) aus Knochenzementteig (36, 86), wobei die Schaftform (1, 51, 151, 251) in ihrem Inneren einen Innenraum (2, 52, 152, 252) aufweist, wobei der Innenraum (2, 52, 152, 252) über eine proximale Öffnung (3, 53, 153) an einer proximalen Seite der Schaftform (1, 51, 151, 251) zugänglich ist und wobei die Schaftform (1, 51, 151, 251) eine proximale Wandung (4, 54, 154) aufweist, die die proximale Öffnung (3, 53, 153) der Schaftform (1, 51, 151, 251) umlaufend begrenzt,
eine Kopfform (5, 55, 105, 155, 205, 255) zum Formen eines Kopfs (40, 90, 190, 240, 290) und eines Halses (44, 94, 194, 244, 294) des Spacers (38, 88, 188, 238, 288) aus Knochenzementteig (36, 86), wobei die Kopfform (5, 55, 105, 155, 205, 255) in ihrem Inneren einen Hohlraum (6, 56, 106, 156, 206, 256) aufweist, wobei der Hohlraum (6, 56, 106, 156, 206, 256) eine kugelflächenförmige Innenfläche (7, 57, 107, 157, 207, 257) zum Formen einer Gleitfläche des Kopfs (40, 90, 190, 240, 290) des Spacers (38, 88, 188, 238, 288) aufweist und der Hohlraum (6, 56, 106, 156, 206, 256) über eine distale Öffnung (8, 58, 108, 158) an einer distalen Seite der Kopfform (5, 55, 105, 155, 205, 255) zugänglich ist, wobei die Kopfform (5, 55, 105, 155, 205, 255) eine distale Wandung (9, 59, 109, 159) aufweist, die die distale Öffnung (8, 58, 108, 158) der Kopfform (5, 55, 105, 155, 205, 255) umlaufend begrenzt,
einen Metallkern (10, 60, 160, 260), wobei der Metallkern (10, 60, 160, 260) einen Schaftteil (12, 62, 162, 262), einen Kopfteil (14, 64, 164, 264) und einen Steg (16, 66, 166, 266) aufweist, wobei der Steg (16, 66, 166, 266) vom Metallkern (10, 60, 160, 260) absteht, wobei der Steg (16, 66, 166, 266) zwischen dem Schaftteil (12, 62, 162, 262) und dem Kopfteil (14, 64, 164, 264) angeordnet ist und wobei der Steg (16, 66, 166, 266) eine proximale Oberfläche (18, 68, 168) und eine distale Oberfläche (20, 70, 170) aufweist,
**dadurch gekennzeichnet, dass**
die Schaftform (1, 51, 151, 251) und das Schaftteil (12, 62, 162, 262) des Metallkerns (10, 60, 160, 260) derart geformt sind, dass das Schaftteil (12, 62, 162, 262) in dem Innenraum (2, 52, 152, 252) der Schaftform (1, 51, 151, 251) angeordnet ist, wenn die proximale Wandung (4, 54, 154) der Schaftform (1, 51, 151, 251) an der distalen Oberfläche (20, 70, 170) des Stegs (16, 66, 166, 266) anliegt, und
die Kopfform (5, 55, 105, 155, 205, 255) und das Kopfteil (14, 64, 164, 264) des Metallkerns (10, 60, 160, 260) derart geformt sind, dass das Kopfteil (14, 64, 164, 264) in dem Hohlraum (6, 56, 106, 156, 206, 256) der Kopfform (5, 55, 105, 155, 205, 255) angeordnet ist, wenn die distale Wandung (9, 59, 109, 159) der Kopfform (5, 55, 105, 155, 205, 255) an der proximalen Oberfläche (18, 68, 168) des Stegs (16, 66, 166, 266) anliegt.

2. Vorrichtung nach Anspruch 1 **dadurch gekennzeichnet, dass**
der Metallkern (10, 60, 160, 260) aus dem Schaftteil (12, 62, 162, 262), dem Kopfteil (14, 64, 164, 264) und dem Steg (16, 66, 166, 266) besteht und/oder der Metallkern (10, 60, 160, 260) einteilig ist.

3. Vorrichtung nach Anspruch 1 oder 2, **dadurch gekennzeichnet, dass** die Schaftform (1, 51, 151, 251) und/oder die Kopfform (5, 55, 105, 155, 205, 255) aus einem Kunststoff besteht oder bestehen, bevorzugt aus zumindest einer Kunststofffolie oder aus spritzgegossenem Kunststoff besteht oder bestehen.

4. Vorrichtung nach einem der vorangehenden Ansprüche, **dadurch gekennzeichnet, dass**
das Schaftteil (12, 62, 162, 262) in dem Innenraum (2, 52, 152, 252) der Schaftform (1, 51, 151, 251) von Innenwänden der Schaftform (1, 51, 151, 251), die den Innenraum (2, 52, 152, 252) begrenzen, beabstandet ist, wenn die proximale Wandung (4, 54, 154) der Schaftform (1, 51, 151, 251) an der distalen Oberfläche (20, 70, 170) des Stegs (16, 66, 166, 266) anliegt, insbesondere flächenbündig an der distalen Oberfläche (20, 70, 170) des Stegs (16, 66, 166, 266) anliegt, und/oder
das Kopfteil (14, 64, 164, 264) in dem Hohlraum (6, 56, 106, 156, 206, 256) der Kopfform (5, 55, 105, 155, 205, 255) von Innenwänden der Kopfform (5, 55, 105, 155, 205, 255), die den Hohlraum (6, 56, 106, 156, 206, 256) begrenzen, beabstandet ist, wenn die distale Wandung (9, 59, 109, 159) der Kopfform (5, 55, 105, 155, 205, 255) an der proximalen Oberfläche (18, 68, 168) des Stegs (16, 66, 166, 266) anliegt, insbesondere flächenbündig an der proximalen Oberfläche (18, 68, 168) des Stegs (16, 66, 166, 266) anliegt.

5. Vorrichtung nach einem der vorangehenden Ansprüche, **dadurch gekennzeichnet, dass**
die Kopfform (5, 55, 105, 155, 205, 255) eine Halsform (28, 78, 128, 178, 228, 278) zum Formen eines den Kopf (40, 90, 190, 240, 290) und den Schaft (42, 92, 192, 242, 292) verbindenden Halses (44, 94, 194, 244, 294) des Spacers (38, 88, 188, 238, 288) aus Knochenzementteig (36, 86) aufweist, wobei die Halsform (28, 78, 128, 178, 228, 278) rohrförmig oder hohlzylinderförmig ist, wobei die distale Öffnung (8, 58, 108, 158) und die distale Wandung (9, 59, 109, 159) der Kopfform (5, 55, 105, 155, 205, 255) beides Teile der Halsform (28, 78, 128, 178, 228, 278) sind und die kugelflächenförmige Innenfläche (7, 57, 107, 157, 207, 257) des Hohlraums (6, 56, 106, 156, 206, 256) kein Teil der Halsform (28, 78, 128, 178, 228, 278) ist, wobei vorzugsweise die Kopfform (5, 55, 105, 155, 205, 255) und das Kopfteil (14, 64, 164, 264) des Metallkerns (10, 60, 160, 260) derart geformt sind, dass das Kopfteil (14, 64, 164, 264) über die Halsform (28, 78, 128, 178, 228, 278) hinaus in den Hohlraum (6, 56, 106, 156, 206, 256) der Kopfform (5, 55, 105, 155, 205, 255) ragt, wenn die distale Wandung (9, 59, 109, 159) an der proximalen Oberfläche (18, 68, 168) des Stegs (16, 66, 166, 266) anliegt.

6. Vorrichtung nach Anspruch 5, **dadurch gekennzeichnet, dass** die Länge der Halsform (28, 78, 128, 178, 228, 278) durch Kürzen der Halsform (28, 78, 128, 178, 228, 278) einstellbar ist, wobei vorzugsweise Sollschnittstellen oder Sollreißstellen an der Halsform (28, 78, 128, 178, 228, 278) angeordnet sind und/oder eine Skalierung außen an der Halsform (28, 78, 128, 178, 228, 278) angeordnet ist.

7. Vorrichtung nach einem der vorangehenden Ansprüche, **dadurch gekennzeichnet, dass**
die proximale Oberfläche (18, 68, 168) des Stegs (16, 66, 166, 266) eine Verschiebung des Metallkerns (10, 60, 160, 260) in der Kopfform (5, 55, 105, 155, 205, 255), insbesondere in der Halsform (28, 78, 128, 178, 228, 278), begrenzt und/oder die distale Oberfläche (20, 70, 170) des Stegs (16, 66, 166, 266) eine Verschiebung des Metallkerns (10, 60, 160, 260) in der Schaftform (1, 51, 151, 251) begrenzt.

8. Vorrichtung nach einem der vorangehenden Ansprüche, **dadurch gekennzeichnet, dass**
am Kopfteil (14, 64, 164, 264) des Metallkerns (10, 60, 160, 260) Zentriereinrichtungen (24, 74, 174, 274) angeordnet sind, wobei die Zentriereinrichtungen (24, 74, 174, 274) von der Oberfläche des Kopfteils (14, 64, 164, 264) abstehen, wobei die Zentriereinrichtungen (24, 74, 174, 274) Innenwände der Kopfform (5, 55, 105, 155, 205, 255) von dem Kopfteil (14, 64, 164, 264) beabstanden, wenn das Kopfteil (14, 64, 164, 264) des Metallkerns (10, 60, 160, 260) in den Hohlraum (6, 56, 106, 156, 206, 256) der Kopfform (5, 55, 105, 155, 205, 255) eingeschoben ist, insbesondere die Halsform (28, 78, 128, 178, 228, 278) der Kopfform (5, 55, 105, 155, 205, 255) von dem Kopfteil (14, 64, 164, 264) beabstanden, wenn das Kopfteil (14, 64, 164, 264) des Metallkerns (10, 60, 160, 260) in den Hohlraum (6, 56, 106, 156, 206, 256) der Kopfform (5, 55, 105, 155, 205, 255) eingeschoben ist, wobei vorzugsweise die Zentriereinrichtungen (24, 74, 174, 274) Zentrierfinnen sind und das Kopfteil (14, 64, 164, 264) des Metallkerns (10, 60, 160, 260) entlang der Zentrierfinnen axial in der Kopfform (5, 55, 105, 155, 205, 255), insbesondere in der Halsform (28, 78, 128, 178, 228, 278), verschiebbar ist, und/oder
am Schaftteil (12, 62, 162, 262) des Metallkerns (10, 60, 160, 260) Abstandhalter (22, 72, 172, 272) angeordnet sind, wobei die Abstandhalter (22, 72, 172, 272) von der Oberfläche des Schaftteils (12, 62, 162, 262) abstehen, wobei die Abstandhalter (22, 72, 172, 272) Innenwände der Schaftform (1, 51, 151, 251) vom Schaftteil (12, 62, 162, 262) des Metallkerns (10, 60, 160, 260) beabstanden, wenn das Schaftteil (12, 62, 162, 262) des Metallkerns (10, 60, 160, 260) in den Innenraum (2, 52, 152, 252) der Schaftform (1, 51, 151, 251) eingeschoben ist.

9. Vorrichtung nach einem der vorangehenden Ansprüche, **dadurch gekennzeichnet, dass**
der Außenumfang des Stegs (16, 66, 166, 266) größer ist als die proximale Öffnung (3, 53, 153) der Schaftform (1, 51, 151, 251) und/oder als die distale Öffnung (8, 58, 108, 158) der Kopfform (5, 55, 105, 155, 205, 255), und/oder
wenigstens eine Entlüftungsöffnung in der Schaftform (1, 51, 151, 251) und/oder der Kopfform (5, 55, 105, 155, 205, 255) angeordnet ist, wobei die wenigstens eine Entlüftungsöffnung den Innenraum (2, 52, 152, 252) der Schaftform (1, 51, 151, 251) und/oder den Hohlraum (6, 56, 106, 156, 206, 256) der Kopfform (5, 55, 105, 155, 205, 255) gasdurchlässig mit der Umgebung der Vorrichtung verbindet, wobei vorzugsweise die wenigstens eine Entlüftungsöffnung für Gase durchlässig ist und für Knochenzementteig (36, 86) undurchlässig ist, insbesondere für Polymethylmethacrylat-Knochenzementteig undurchlässig ist.

10. Vorrichtung nach einem der vorangehenden Ansprüche, **dadurch gekennzeichnet, dass**
die Vorrichtung ein in zumindest einem keimdichten Zementbehälter verpacktes Zementpulver und eine in zumindest einem keimdichten und flüssigkeitsdichten Monomerflüssigkeitsbehälter verpackte Monomerflüssigkeit aufweist, wobei vorzugsweise
die Vorrichtung zumindest eine Knochenzementkartusche (132) zum Mischen des Zementpulvers mit der Monomerflüssigkeit und zum Austragen von gemischtem Knochenzementteig (36, 86) aus der Knochenzementkartusche (132) heraus aufweist, bevorzugt eine Knochenzementkartusche (132) zum Mischen von Polymethylmethacrylat-Knochenzement-Ausgangskomponenten und zum Austragen von gemischtem Polymethylmethacrylat-Knochenzementteig aus der Knochenzementkartusche (132) heraus aufweist, wobei besonders bevorzugt die zumindest eine Knochenzementkartusche (132) den zumindest einen Zementbehälter aufweist und den zumindest einen Monomerflüssigkeitsbehälter enthält, wobei der zumindest eine Zementbehälter und der zumindest eine Monomerflüssigkeitsbehälter in voneinander getrennten Bereichen angeordnet sind.

11. Set zum Herstellen unterschiedlicher Spacer (38, 88, 188, 238, 288) durch Aushärten von Knochenzementteig (36, 86), wobei die Spacer (38, 88, 188, 238, 288) dazu vorgesehen sind, im medizinischen Bereich ein Gelenk oder einen Teil eines Gelenks umfassend eine artikulierende Oberfläche eines Kopfs des Gelenks temporär zu ersetzen, insbesondere eines Hüftgelenks oder eines Schultergelenks temporär zu ersetzen, das Set aufweisend mindestens eine Vorrichtung nach einem der vorangehenden Ansprüche, wobei das Set zumindest zwei Kopfformen (5, 55, 105, 155, 205, 255) mit unterschiedlichem Durchmesser der kugelflächenförmigen Innenflächen (7, 57, 107, 157, 207, 257) aufweist und/oder zumindest zwei Schaftformen (1, 51, 151, 251) mit in distaler Richtung unterschiedlich lang gestreckten Hohlräumen (6, 56, 106, 156, 206, 256) oder unterschiedlichen Innendurchmessern der Hohlräume (6, 56, 106, 156, 206, 256) aufweist.

12. Set nach Anspruch 11, **dadurch gekennzeichnet, dass**
das Set zumindest zwei Metallkerne (10, 60, 160, 260) mit unterschiedlicher Länge des Kopfteils (14, 64, 164, 264) und/oder des Schaftteils (12, 62, 162, 262) aufweist.

13. Verfahren zur Herstellung eines Spacers (38, 88, 188, 238, 288) zum temporären Ersetzen eines Gelenks oder eines Teils eines Gelenks, insbesondere eines Hüftgelenks oder eines Schultergelenks, umfassend eine artikulierende Oberfläche des Gelenks, wobei das Verfahren mit einer Vorrichtung nach einem der Ansprüche 1 bis 10 oder mit einem Set nach einem der Ansprüche 11 oder 12 durchgeführt wird, das Verfahren aufweisend die folgenden Schritte:
A) Einfüllen eines Knochenzementteigs (36, 86) in den Innenraum (2, 52, 152, 252) der Schaftform (1, 51, 151, 251) und Einfüllen eines Knochenzementteigs (36, 86) in den Hohlraum (6, 56, 106, 156, 206, 256) der Kopfform (5, 55, 105, 155, 205, 255);
B) Einschieben des Kopfteils (14, 64, 164, 264) des Metallkerns (10, 60, 160, 260) in den mit Knochenzementteig (36, 86) gefüllten Hohlraum (6, 56, 106, 156, 206, 256) der Kopfform (5, 55, 105, 155, 205, 255) unter Verdrängung des darin enthaltenen Knochenzementteigs (36, 86), bis die distale Wandung (9, 59, 109, 159) der Kopfform (5, 55, 105, 155, 205, 255) an der proximalen Oberfläche (18, 68, 168) des Stegs (16, 66, 166, 266) des Metallkerns (10, 60, 160, 260) anliegt, und Einschieben des Schaftteils (12, 62, 162, 262) des Metallkerns (10, 60, 160, 260) in den mit Knochenzementteig (36, 86) gefüllten Innenraum (2, 52, 152, 252) der Schaftform (1, 51, 151, 251) unter Verdrängung des darin enthaltenen Knochenzementteigs (36, 86), bis die proximale Wandung (4, 54, 154) der Schaftform (1, 51, 151, 251) an der distalen Oberfläche (20, 70, 170) des Stegs (16, 66, 166, 266) des Metallkerns (10, 60, 160, 260) anliegt;
C) Aushärten des Knochenzementteigs (36, 86) in der Kopfform (5, 55, 105, 155, 205, 255) und der Schaftform (1, 51, 151, 251); und
D) Entnehmen des so geformten und ausgehärteten Spacers (38, 88, 188, 238, 288) aus der Kopfform (5, 55, 105, 155, 205, 255) und der Schaftform (1, 51, 151, 251).

14. Verfahren nach Anspruch 13, **dadurch gekennzeichnet, dass**
vor Schritt A) eine Halsform (28, 78, 128, 178, 228, 278) der Kopfform (5, 55, 105, 155, 205, 255) entsprechend einer gewünschten Länge des Halses (44, 94, 194, 244, 294) des herzustellenden Spacers (38, 88, 188, 238, 288) gekürzt wird, eine Kopfform (5, 55, 105, 155, 205, 255) mit einem passenden Innendurchmesser der kugelflächenförmigen Innenfläche (7, 57, 107, 157, 207, 257) passend zur gewünschten Form des Kopf (40, 90, 190, 240, 290) des herzustellenden Spacers (38, 88, 188, 238, 288) ausgewählt wird, eine Schaftform (1, 51, 151, 251) mit einem zur gewünschten Form des Schafts (42, 92, 192, 242, 292) des herzustellenden Spacers (38, 88, 188, 238, 288) passenden Innenraum (2, 52, 152, 252) ausgewählt wird und/oder ein Metallkern (10, 60, 160, 260) mit zu der gewünschten Form des Schafts (42, 92, 192, 242, 292) des herzustellenden Spacers (38, 88, 188, 238, 288) passenden Abmessungen ausgewählt wird, und/oder
vor Schritt A) der homogener Knochenzementteig (36, 86) durch Mischen einer Monomerflüssigkeit und einem Zementpulver hergestellt wird, wobei bevorzugt in Schritt A) der gemischte Knochenzementteig (36, 86) mit einer Knochenzementkartusche in den Innenraum (2, 52, 152, 252) der Schaftform (1, 51, 151, 251) und in den Hohlraum (6, 56, 106, 156, 206, 256) der Kopfform (5, 55, 105, 155, 205, 255) gepresst wird, wobei besonders bevorzugt dabei die Luft aus dem Innenraum (2, 52, 152, 252) der Schaftform (1, 51, 151, 251) und aus dem Hohlraum (6, 56, 106, 156, 206, 256) der Kopfform (5, 55, 105, 155, 205, 255) verdrängt wird.

15. Verfahren nach einem der Ansprüche 13 oder 14, **dadurch gekennzeichnet, dass** in Schritt B) das Kopfteil (14, 64, 164, 264) des Metallkerns (10, 60, 160, 260) mit Hilfe von Zentriereinrichtungen (24, 74, 174, 274), insbesondere mit Hilfe von Zentrierfinnen, die von der Oberfläche des Kopfteils (14, 64, 164, 264) abstehen und die beim Einschieben des Kopfteils (14, 64, 164, 264) in den Hohlraum (6, 56, 106, 156, 206, 256) der Kopfform (5, 55, 105, 155, 205, 255) an Innenwänden des Hohlraums (6, 56, 106, 156, 206, 256) der Kopfform (5, 55, 105, 155, 205, 255) anliegen, in der Kopfform (5, 55, 105, 155, 205, 255) zentriert werden, und/oder
in Schritt B) das Schaftteil (12, 62, 162, 262) des Metallkerns (10, 60, 160, 260) mit Hilfe von Abstandhaltern (22, 72, 172, 272), die von der Oberfläche des Schaftteils (12, 62, 162, 262) abstehen und die beim Einschieben des Schaftteils (12, 62, 162, 262) in den Innenraum (2, 52, 152, 252) der Schaftform (1, 51, 151, 251) an Innenwänden des Innenraums (2, 52, 152, 252) der Schaftform (1, 51, 151, 251) anliegen, in der Schaftform (1, 51, 151, 251) zentriert werden.

## Claims

1. A device for producing a spacer (38, 88, 188, 238, 288) by curing bone cement paste (36, 86), wherein the spacer (38, 88, 188, 238, 288) is provided in the medical field for temporarily replacing a joint or part of a joint comprising an articulating surface of a head of the joint, in particular for temporarily replacing a hip joint or a shoulder joint, the device having
a stem mold (1, 51, 151, 251) for shaping a stem (42, 92, 192, 242, 292) of the spacer (38, 88, 188, 238, 288) from bone cement paste (36, 86), wherein the stem mold (1, 51, 151, 251) has in the interior thereof an inner space (2, 52, 152, 252), wherein the inner space (2, 52, 152, 252) is accessible via a proximal opening (3, 53, 153) on a proximal side of the stem mold (1, 51, 151, 251) and wherein the stem mold (1, 51, 151, 251) has a proximal wall (4, 54, 154) which peripherally delimits the proximal opening (3, 53, 153) of the stem mold (1, 51, 151, 251),
a head mold (5, 55, 105, 155, 205, 255) for shaping a head (40, 90, 190, 240, 290) and a neck (44, 94, 194, 244, 294) of the spacer (38, 88, 188, 238, 288) from bone cement paste (36, 86), wherein the head mold (5, 55, 105, 155, 205, 255) has in the interior thereof a hollow space (6, 56, 106, 156, 206, 256), wherein the hollow space (6, 56, 106, 156, 206, 256) has a spherical surface-shaped inner surface (7, 57, 107, 157, 207, 257) for shaping a sliding surface of the head (40, 90, 190, 240, 290) of the spacer (38, 88, 188, 238, 288) and the hollow space (6, 56, 106, 156, 206, 256) is accessible via a distal opening (8, 58, 108, 158) on a distal side of the head mold (5, 55, 105, 155, 205, 255), wherein the head mold (5, 55, 105, 155, 205, 255) has a distal wall (9, 59, 109, 159) which peripherally delimits the distal opening (8, 58, 108, 158) of the head mold (5, 55, 105, 155, 205, 255),
a metal core (10, 60, 160, 260), wherein the metal core (10, 60, 160, 260) has a stem part (12, 62, 162, 262), a head part (14, 64, 164, 264) and a flange (16, 66, 166, 266), wherein the flange (16, 66, 166, 266) projects out from the metal core (10, 60, 160, 260), wherein the flange (16, 66, 166, 266) is arranged between the stem part (12, 62, 162, 262) and the head part (14, 64, 164, 264) and wherein the flange (16, 66, 166, 266) has a proximal surface (18, 68, 168) and a distal surface (20, 70, 170), **characterized in that**
the stem mold (1, 51, 151, 251) and the stem part (12, 62, 162, 262) of the metal core (10, 60, 160, 260) are shaped such that the stem part (12, 62, 162, 262) is arranged in the inner space (2, 52, 152, 252) of the stem mold (1, 51, 151, 251), when the proximal wall (4, 54, 154) of the stem mold (1, 51, 151, 251) is resting against the distal surface (20, 70, 170) of the flange (16, 66, 166, 266), and
the head mold (5, 55, 105, 155, 205, 255) and the head part (14, 64, 164, 264) of the metal core (10, 60, 160, 260) are shaped such that the head part (14, 64, 164, 264) is arranged in the hollow space (6, 56, 106, 156, 206, 256) of the head mold (5, 55, 105, 155, 205, 255), when the distal wall (9, 59, 109, 159) of the head mold (5, 55, 105, 155, 205, 255) is resting against the proximal surface (18, 68, 168) of the flange (16, 66, 166, 266).

2. The device according to Claim 1, **characterized in that**
the metal core (10, 60, 160, 260) consists of the stem part (12, 62, 162, 262), the head part (14, 64, 164, 264) and the flange (16, 66, 166, 266) and/or the metal core (10, 60, 160, 260) is one-part.

3. The device according to Claim 1 or 2, **characterized in that**
the stem mold (1, 51, 151, 251) and/or the head mold (5, 55, 105, 155, 205, 255) consists or consist of a plastics material, preferably of at least one plastics film or of an injection-molded plastics material.

4. The device according to any one of the preceding claims, **characterized in that**
the stem part (12, 62, 162, 262) is spaced in the inner space (2, 52, 152, 252) of the stem mold (1, 51, 151, 251) from inner walls of the stem mold (1, 51, 151, 251) which delimit the inner space (2, 52, 152, 252), when the proximal wall (4, 54, 154) of the stem mold (1, 51, 151, 251) is resting against the distal surface (20, 70, 170) of the flange (16, 66, 166, 266), in particular is resting flush against the distal surface (20, 70, 170) of the flange (16, 66, 166, 266), and/or
the head part (14, 64, 164, 264) is spaced in the hollow space (6, 56, 106, 156, 206, 256) of the head mold (5, 55, 105, 155, 205, 255) from inner walls of the head mold (5, 55, 105, 155, 205, 255) which delimit the hollow space (6, 56, 106, 156, 206, 256), when the distal wall (9, 59, 109, 159) of the head mold (5, 55, 105, 155, 205, 255) is resting against the proximal surface (18, 68, 168) of the flange (16, 66, 166, 266), in particular is resting flush against the proximal surface (18, 68, 168) of the flange (16, 66, 166, 266).

5. The device according to any one of the preceding claims, **characterized in that** the head mold (5, 55, 105, 155, 205, 255) has a neck mold (28, 78, 128, 178, 228, 278) for shaping a neck (44, 94, 194, 244, 294) of the spacer (38, 88, 188, 238, 288), which neck connects the head (40, 90, 190, 240, 290) and the stem (42, 92, 192, 242, 292), from bone cement paste (36, 86), wherein the neck mold (28, 78, 128, 178, 228, 278) is tubular or hollow-cylindrical, wherein the distal opening (8, 58, 108, 158) and the distal wall (9, 59, 109, 159) of the head mold (5, 55, 105, 155, 205, 255) are both parts of the neck mold (28, 78, 128, 178, 228, 278) and the spherical surface-shaped inner surface (7, 57, 107, 157, 207, 257) of the hollow space (6, 56, 106, 156, 206, 256) is not part of the neck mold (28, 78, 128, 178, 228, 278), wherein preferably the head mold (5, 55, 105, 155, 205, 255) and the head part (14, 64, 164, 264) of the metal core (10, 60, 160, 260) are shaped such that the head part (14, 64, 164, 264) protrudes beyond the neck mold (28, 78, 128, 178, 228, 278) into the hollow space (6, 56, 106, 156, 206, 256) of the head mold (5, 55, 105, 155, 205, 255), when the distal wall (9, 59, 109, 159) is resting against the proximal surface (18, 68, 168) of the flange (16, 66, 166, 266).

6. The device according to Claim 5, **characterized in that**
the length of the neck mold (28, 78, 128, 178, 228, 278) is adjustable by shortening the neck mold (28, 78, 128, 178, 228, 278), wherein, preferably, predetermined cutting or tearing points are arranged on the neck mold (28, 78, 128, 178, 228, 278) and/or a scale is arranged externally on the neck mold (28, 78, 128, 178, 228, 278).

7. The device according to any one of the preceding claims, **characterized in that**
the proximal surface (18, 68, 168) of the flange (16, 66, 166, 266) limits displacement of the metal core (10, 60, 160, 260) in the head mold (5, 55, 105, 155, 205, 255), in particular in the neck mold (28, 78, 128, 178, 228, 278), and/or the distal surface (20, 70, 170) of the flange (16, 66, 166, 266) limits displacement of the metal core (10, 60, 160, 260) in the stem mold (1, 51, 151, 251).

8. The device according to any one of the preceding claims, **characterized in that**
centering means (24, 74, 174, 274) are arranged on the head part (14, 64, 164, 264) of the metal core (10, 60, 160, 260), wherein the centering means (24, 74, 174, 274) project out from the surface of the head part (14, 64, 164, 264), wherein the centering means (24, 74, 174, 274) space inner walls of the head mold (5, 55, 105, 155, 205, 255) from the head part (14, 64, 164, 264), when the head part (14, 64, 164, 264) of the metal core (10, 60, 160, 260) is pushed into the hollow space (6, 56, 106, 156, 206, 256) of the head mold (5, 55, 105, 155, 205, 255), in particular the neck mold (28, 78, 128, 178, 228, 278) of the head mold (5, 55, 105, 155, 205, 255) is spaced from the head part (14, 64, 164, 264), when the head part (14, 64, 164, 264) of the metal core (10, 60, 160, 260) is pushed into the hollow space (6, 56, 106, 156, 206, 256) of the head mold (5, 55, 105, 155, 205, 255), wherein the centering means (24, 74, 174, 274) are preferably centering fins and the head part (14, 64, 164, 264) of the metal core (10, 60, 160, 260) is axially displaceable along the centering fins in the head mold (5, 55, 105, 155, 205, 255), in particular is displaceable in the neck mold (28, 78, 128, 178, 228, 278), and/or
spacing pieces (22, 72, 172, 272) are arranged on the stem part (12, 62, 162, 262) of the metal core (10, 60, 160, 260), wherein the spacing pieces (22, 72, 172, 272) project out from the surface of the stem part (12, 62, 162, 262), wherein the spacing pieces (22, 72, 172, 272) space inner walls of the stem mold (1, 51, 151, 251) from the stem part (12, 62, 162, 262) of the metal core (10, 60, 160, 260), when the stem part (12, 62, 162, 262) of the metal core (10, 60, 160, 260) is pushed into the inner space (2, 52, 152, 252) of the stem mold (1, 51, 151, 251).

9. The device according to any one of the preceding claims, **characterized in that** the outer circumference of the flange (16, 66, 166, 266) is larger than at least one of the proximal opening (3, 53, 153) of the stem mold (1, 51, 151, 251) and the distal opening (8, 58, 108, 158) of the head mold (5, 55, 105, 155, 205, 255), and/or at least one vent opening is arranged in at least one of the stem mold (1, 51, 151, 251) and the head mold (5, 55, 105, 155, 205, 255), wherein the at least one vent opening gas-permeably connects at least one of the inner space (2, 52, 152, 252) of the stem mold (1, 51, 151, 251) and the hollow space (6, 56, 106, 156, 206, 256) of the head mold (5, 55, 105, 155, 205, 255) with the surroundings of the device, wherein the at least one vent opening is preferably permeable to gases and impermeable to bone cement paste (36, 86), in particular is impermeable to polymethyl methacrylate bone cement paste.

10. The device according to any one of the preceding claims, **characterized in that**
the device has a cement powder packaged in at least one microbe-proof cement container and a monomer liquid packaged in at least one microbe-proof and liquid-tight monomer liquid container, wherein preferably
the device has at least one bone cement cartridge (132) for mixing the cement powder with the monomer liquid and for delivering mixed bone cement paste (36, 86) from the bone cement cartridge (132) and preferably has a bone cement cartridge (132) for mixing polymethyl methacrylate bone cement starting components and for delivering mixed polymethyl methacrylate bone cement paste from the bone cement cartridge (132), wherein particularly preferably the at least one bone cement cartridge (132) has the at least one cement container and contains the at least one monomer liquid container, wherein the at least one cement container and the at least one monomer liquid container are arranged in mutually separate regions.

11. A set for producing different spacers (38, 88, 188, 238, 288) by curing bone cement paste (36, 86), wherein the spacers (38, 88, 188, 238, 288) are provided in the medical field for temporarily replacing a joint or part of a joint comprising an articulating surface of a head of the joint, in particular for temporarily replacing a hip joint or a shoulder joint, the set having at least one device according to one of the preceding claims, wherein the set has at least two head molds (5, 55, 105, 155, 205, 255) with different diameters of the spherical surface-shaped inner surfaces (7, 57, 107, 157, 207, 257) and/or at least two stem molds (1, 51, 151, 251) with hollow spaces (6, 56, 106, 156, 206, 256) of different lengths in the distal direction or different internal diameters of the hollow spaces (6, 56, 106, 156, 206, 256).

12. The set according to Claim 11, **characterized in that**
the set has at least two metal cores (10, 60, 160, 260) with a different length of the head part (14, 64, 164, 264) and/or stem part (12, 62, 162, 262).

13. A method for producing a spacer (38, 88, 188, 238, 288) for temporarily replacing a joint or part of a joint, in particular a hip joint or a shoulder joint, comprising an articulating surface of the joint, wherein the method is carried out with the device according to any one of Claims 1 to 10 or with a set according to either one of Claims 11 or 12, the method having the following steps:
A) introducing a bone cement paste (36, 86) into the inner space (2, 52, 152, 252) of the stem mold (1, 51, 151, 251) and introducing a bone cement paste (36, 86) into the hollow space (6, 56, 106, 156, 206, 256) of the head mold (5, 55, 105, 155, 205, 255);
B) pushing the head part (14, 64, 164, 264) of the metal core (10, 60, 160, 260) into the hollow space (6, 56, 106, 156, 206, 256), filled with bone cement paste (36, 86), of the head mold (5, 55, 105, 155, 205, 255), so displacing the bone cement paste (36, 86) contained therein, until the distal wall (9, 59, 109, 159) of the head mold (5, 55, 105, 155, 205, 255) is resting against the proximal surface (18, 68, 168) of the flange (16, 66, 166, 266) of the metal core (10, 60, 160, 260), and pushing the stem part (12, 62, 162, 262) of the metal core (10, 60, 160, 260) into the inner space (2, 52, 152, 252), filled with bone cement paste (36, 86), of the stem mold (1, 51, 151, 251), so displacing the bone cement paste (36, 86) contained therein, until the proximal wall (4, 54, 154) of the stem mold (1, 51, 151, 251) is resting against the distal surface (20, 70, 170) of the flange (16, 66, 166, 266) of the metal core (10, 60, 160, 260);
C) curing the bone cement paste (36, 86) in the head mold (5, 55, 105, 155, 205, 255) and the stem mold (1, 51, 151, 251); and
D) removing the resultant shaped and cured spacer (38, 88, 188, 238, 288) from the head mold (5, 55, 105, 155, 205, 255) and the stem mold (1, 51, 151, 251).

14. The method according to Claim 13, **characterized in that**,
before step A), a neck mold (28, 78, 128, 178, 228, 278) of the head mold (5, 55, 105, 155, 205, 255) is shortened in accordance with a desired length of the neck (44, 94, 194, 244, 294) of the spacer (38, 88, 188, 238, 288) to be produced, a head mold (5, 55, 105, 155, 205, 255) with a matching internal diameter of the spherical surface-shaped inner surface (7, 57, 107, 157, 207, 257) is selected to match the desired shape of the head (40, 90, 190, 240, 290) of the spacer (38, 88, 188, 238, 288) to be produced, a stem mold (1, 51, 151, 251) with an inner space (2, 52, 152, 252) matching the desired shape of the stem (42, 92, 192, 242, 292) of the spacer (38, 88, 188, 238, 288) to be produced is selected and/or a metal core (10, 60, 160, 260) with dimensions matching the desired shape of the stem (42, 92, 192, 242, 292) of the spacer (38, 88, 188, 238, 288) to be produced is selected, and/or
before step A), the homogeneous bone cement paste (36, 86) is produced by mixing a monomer liquid and a cement powder, wherein preferably in step A) the mixed bone cement paste (36, 86) is pressed with a bone cement cartridge into the inner space (2, 52, 152, 252) of the stem mold (1, 51, 151, 251) and into the hollow space (6, 56, 106, 156, 206, 256) of the head mold (5, 55, 105, 155, 205, 255), wherein particularly preferably in so doing the air is expelled from the inner space (2, 52, 152, 252) of the stem mold (1, 51, 151, 251) and from the hollow space (6, 56, 106, 156, 206, 256) of the head mold (5, 55, 105, 155, 205, 255).

15. The method according to one of Claims 13 or 14, **characterized in that**,
in step B), the head part (14, 64, 164, 264) of the metal core (10, 60, 160, 260) is centered in the head mold (5, 55, 105, 155, 205, 255) with the assistance of centering means (24, 74, 174, 274), in particular with the assistance of centering fins, which project out from the surface of the head part (14, 64, 164, 264) and which, when the head part (14, 64, 164, 264) is pushed into the hollow space (6, 56, 106, 156, 206, 256) of the head mold (5, 55, 105, 155, 205, 255), rest against inner walls of the hollow space (6, 56, 106, 156, 206, 256) of the head mold (5, 55, 105, 155, 205, 255), and/or in step B), the stem part (12, 62, 162, 262) of the metal core (10, 60, 160, 260) is centered in the stem mold (1, 51, 151, 251) with the assistance of spacing pieces (22, 72, 172, 272), which project out from the surface of the stem part (12, 62, 162, 262) and which, when the stem part (12, 62, 162, 262) is pushed into the inner space (2, 52, 152, 252) of the stem mold (1, 51, 151, 251), rest against inner walls of the inner space (2, 52, 152, 252) of the stem mold (1, 51, 151, 251).

## Revendications

1. Dispositif de fabrication d'un espaceur (38, 88, 188, 238, 288) par durcissement de pâte de ciment osseux (36, 86), l'espaceur (38, 88, 188, 238, 288) étant prévu pour remplacer temporairement, dans le domaine médical, une articulation ou une partie d'une articulation comprenant une surface articulée d'une tête de l'articulation, en particulier pour remplacer temporairement une articulation de la hanche ou une articulation de l'épaule, le dispositif présentant un moule de tige (1, 51, 151, 251) permettant de mouler une tige (42, 92, 192, 242, 292) de l'espaceur (38, 88, 188, 238, 288) à partir de pâte de ciment osseux (36, 86), le moule de tige (1, 51, 151, 251) présentant un espace intérieur (2, 52, 152, 252) dans son intérieur, l'espace intérieur (2, 52, 152, 252) étant accessible par l'intermédiaire d'une ouverture proximale (3, 53, 153) sur une face proximale du moule de tige (1, 51, 151, 251) et le moule de tige (1, 51, 151, 251) présentant une paroi proximale (4, 54, 154) qui délimite de manière circonférentielle l'ouverture proximale (3, 53, 153) du moule de tige (1, 51, 151, 251), un moule de tête (5, 55, 105, 155, 205, 255) permettant de mouler une tête (40, 90, 190, 240, 290) et un col (44, 94, 194, 244, 294) de l'espaceur (38, 88, 188, 238, 288) à partir de pâte de ciment osseux (36, 86), le moule de tête (5, 55, 105, 155, 205, 255) présentant une cavité (6, 56, 106, 156, 206, 256) dans son intérieur, la cavité (6, 56, 106, 156, 206, 256) présentant une surface intérieure sphérique (7, 57, 107, 157, 207, 257) permettant de mouler une surface de glissement de la tête (40, 90, 190, 240, 290) de l'espaceur (38, 88, 188, 238, 288) et la cavité (6, 56, 106, 156, 206, 256) étant accessible par l'intermédiaire d'une ouverture distale (8, 58, 108, 158) sur une face distale du moule de tête (5, 55, 105, 155, 205, 255), le moule de tête (5, 55, 105, 155, 205, 255) présentant une paroi distale (9, 59, 109, 159) qui délimite de manière circonférentielle l'ouverture distale (8, 58, 108, 158) du moule de tête (5, 55, 105, 155, 205, 255),
un noyau métallique (10, 60, 160, 260), le noyau métallique (10, 60, 160, 260) présentant une partie de tige (12, 62, 162, 262), une partie de tête (14, 64, 164, 264) et une nervure (16, 66, 166, 266), la nervure (16, 66, 166, 266) faisant saillie depuis le noyau métallique (10, 60, 160, 260), la nervure (16, 66, 166, 266) étant disposée entre la partie de tige (12, 62, 162, 262) et la partie de tête (14, 64, 164, 264) et la nervure (16, 66, 166, 266) présentant une surface proximale (18, 68, 168) et une surface distale (20, 70, 170), **caractérisé en ce que** le moule de tige (1, 51, 151, 251) et la partie de tige (12, 62, 162, 262) du noyau métallique (10, 60, 160, 260) sont formés de telle sorte que la partie de tige (12, 62, 162, 262) est disposée dans l'espace intérieur (2, 52, 152, 252) du moule de tige (1, 51, 151, 251) lorsque la paroi proximale (4, 54, 154) du moule de tige (1, 51, 151, 251) repose contre la surface distale (20, 70, 170) de la nervure (16, 66, 166, 266), et le moule de tête (5, 55, 105, 155, 205, 255) et la partie de tête (14, 64, 164, 264) du noyau métallique (10, 60, 160, 260) sont formés de telle sorte que la partie de tête (14, 64, 164, 264) est disposée dans la cavité (6, 56, 106, 156, 206, 256) du moule de tête (5, 55, 105, 155, 205, 255) lorsque la paroi distale (9, 59, 109, 159) du moule de tête (5, 55, 105, 155, 205, 255) repose contre la surface proximale (18, 68, 168) de la nervure (16, 66, 166, 266).

2. Dispositif selon la revendication 1, **caractérisé en ce que** le noyau métallique (10, 60, 160, 260) est constitué de la partie de tige (12, 62, 162, 262), de la partie de tête (14, 64, 164, 264) et de la nervure (16, 66, 166, 266) et/ou le noyau métallique (10, 60, 160, 260) est monobloc.

3. Dispositif selon la revendication 1 ou 2, **caractérisé en ce que** le moule de tige (1, 51, 151, 251) et/ou le moule de tête (5, 55, 105, 155, 205, 255) sont constitués d'une matière plastique, de préférence sont constitués d'au moins un film en matière plastique ou d'une matière plastique moulée par injection.

4. Dispositif selon l'une des revendications précédentes, **caractérisé en ce que** la partie de tige (12, 62, 162, 262) est espacée de parois intérieures du moule de tige (1, 51, 151, 251) dans l'espace intérieur (2, 52, 152, 252) du moule de tige (1, 51, 151, 251), lesquelles parois intérieures délimitent l'espace intérieur (2, 52, 152, 252), lorsque la paroi proximale (4, 54, 154) du moule de tige (1, 51, 151, 251) repose contre la surface distale (20, 70, 170) de la nervure (16, 66, 166, 266), en particulier repose contre la surface distale (20, 70, 170) de la nervure (16, 66, 166, 266) de manière à l'affleurer et/ou la partie de tête (14, 64, 164, 264) est espacée de parois intérieures du moule de tête (5, 55, 105, 155, 205, 255) dans la cavité (6, 56, 106, 156, 206, 256) du moule de tête (5, 55, 105, 155, 205, 255), lesquelles parois intérieures délimitent la cavité (6, 56, 106, 156, 206, 256), lorsque la paroi distale (9, 59, 109, 159) du moule de tête (5, 55, 105, 155, 205, 255) repose contre la surface proximale (18, 68, 168) de la nervure (16, 66, 166, 266), en particulier repose contre la surface proximale (18, 68, 168) de la nervure (16, 66, 166, 266) de manière à l'affleurer.

5. Dispositif selon l'une des revendications précédentes, **caractérisé en ce que** le moule de tête (5, 55, 105, 155, 205, 255) présente un moule de col (28, 78, 128, 178, 228, 278) permettant de mouler un col (44, 94, 194, 244, 294) de l'espaceur (38, 88, 188, 238, 288) reliant la tête (40, 90, 190, 240, 290) et la tige (42, 92, 192, 242, 292) à partir de pâte de ciment osseux (36, 86), le moule de col (28, 78, 128, 178, 228, 278) étant en forme de tube ou de cylindre creux, l'ouverture distale (8, 58, 108, 158) et la paroi distale (9, 59, 109, 159) du moule de tête (5, 55, 105, 155, 205, 255) faisant toutes deux partie du moule de col (28, 78, 128, 178, 228, 278) et la surface intérieure sphérique (7, 57, 107, 157, 207, 257) de la cavité (6, 56, 106, 156, 206, 256) ne faisant pas partie du moule de col (28, 78, 128, 178, 228, 278), de préférence le moule de tête (5, 55, 105, 155, 205, 255) et la partie de tête (14, 64, 164, 264) du noyau métallique (10, 60, 160, 260) étant formés de telle sorte que la partie de tête (14, 64, 164, 264) fait saillie depuis le moule de col (28, 78, 128, 178, 228, 278) dans la cavité (6, 56, 106, 156, 206, 256) du moule de tête (5, 55, 105, 155, 205, 255) lorsque la paroi distale (9, 59, 109, 159) repose contre la surface proximale (18, 68, 168) de la nervure (16, 66, 166, 266).

6. Dispositif selon la revendication 5, **caractérisé en ce que** la longueur du moule de col (28, 78, 128, 178, 228, 278) peut être réglée par raccourcissement du moule de col (28, 78, 128, 178, 228, 278), de préférence des interfaces de consigne ou des emplacements de rupture de consigne étant disposés sur le moule de col (28, 78, 128, 178, 228, 278) et/ou une graduation étant disposée sur le moule de col (28, 78, 128, 178, 228, 278) à l'extérieur.

7. Dispositif selon l'une des revendications précédentes, **caractérisé en ce que** la surface proximale (18, 68, 168) de la nervure (16, 66, 166, 266) limite un déplacement du noyau métallique (10, 60, 160, 260) dans le moule de tête (5, 55, 105, 155, 205, 255), en particulier dans le moule de col (28, 78, 128, 178, 228, 278), et/ou la surface distale (20, 70, 170) de la nervure (16, 66, 166, 266) limite un déplacement du noyau métallique (10, 60, 160, 260) dans le moule de tige (1, 51, 151, 251).

8. Dispositif selon l'une des revendications précédentes, **caractérisé en ce que** des appareils de centrage (24, 74, 174, 274) sont disposés sur la partie de tête (14, 64, 164, 264) du noyau métallique (10, 60, 160, 260), les appareils de centrage (24, 74, 174, 274) faisant saillie depuis la surface de la partie de tête (14, 64, 164, 264), les appareils de centrage (24, 74, 174, 274) séparant des parois intérieures du moule de tête (5, 55, 105, 155, 205, 255) de la partie de tête (14, 64, 164, 264) lorsque la partie de tête (14, 64, 164, 264) du noyau métallique (10, 60, 160, 260) est insérée dans la cavité (6, 56, 106, 156, 206, 256) du moule de tête (5, 55, 105, 155, 205, 255), en particulier séparant le moule de col (28, 78, 128, 178, 228, 278) du moule de tête (5, 55, 105, 155, 205, 255) de la partie de tête (14, 64, 164, 264) lorsque la partie de tête (14, 64, 164, 264) du noyau métallique (10, 60, 160, 260) est insérée dans la cavité (6, 56, 106, 156, 206, 256) du moule de tête (5, 55, 105, 155, 205, 255), de préférence les appareils de centrage (24, 74, 174, 274) étant des ailettes de centrage et la partie de tête (14, 64, 164, 264) du noyau métallique (10, 60, 160, 260) pouvant être déplacée axialement le long des ailettes de centrage dans le moule de tête (5, 55, 105, 155, 205, 255), en particulier dans le moule de col (28, 78, 128, 178, 228, 278), et/ou des entretoises (22, 72, 172, 272) étant disposées sur la partie de tige (12, 62, 162, 262) du noyau métallique (10, 60, 160, 260), les entretoises (22, 72, 172, 272) faisant saillie depuis la surface de la partie de tige (12, 62, 162, 262), les entretoises (22, 72, 172, 272) séparant des parois intérieures du moule de tige (1, 51, 151, 251) de la partie de tige (12, 62, 162, 262) du noyau métallique (10, 60, 160, 260) lorsque la partie de tige (12, 62, 162, 262) du noyau métallique (10, 60, 160, 260) est insérée dans l'espace intérieur (2, 52, 152, 252) du moule de tige (1, 51, 151, 251).

9. Dispositif selon l'une des revendications précédentes, **caractérisé en ce que** la circonférence extérieure de la nervure (16, 66, 166, 266) est supérieure à l'ouverture proximale (3, 53, 153) du moule de tige (1, 51, 151, 251) et/ou à l'ouverture distale (8, 58, 108, 158) du moule de tête (5, 55, 105, 155, 205, 255) et/ou au moins une ouverture de ventilation est disposée dans le moule de tige (1, 51, 151, 251) et/ou le moule de tête (5, 55, 105, 155, 205, 255), l'au moins une ouverture de ventilation reliant de manière perméable aux gaz l'espace intérieur (2, 52, 152, 252) du moule de tige (1, 51, 151, 251) et/ou la cavité (6, 56, 106, 156, 206, 256) du moule de tête (5, 55, 105, 155, 205, 255) à l'environnement du dispositif, de préférence l'au moins une ouverture de ventilation étant perméable aux gaz et étant imperméable à la pâte de ciment osseux (36, 86), en particulier étant imperméable à la pâte de ciment osseux en polyméthacrylate de méthyle.

10. Dispositif selon l'une des revendications précédentes, **caractérisé en ce que** le dispositif présente une poudre de ciment emballée dans au moins un réservoir de ciment étanche aux germes et un liquide monomère emballé dans au moins un réservoir de liquide monomère étanche aux germes et aux liquides, de préférence le dispositif présentant au moins une cartouche de ciment osseux (132) pour le mélange de la poudre de ciment avec le liquide monomère et pour l'évacuation de pâte de ciment osseux (36, 86) mélangée hors de la cartouche de ciment osseux (132), plus préférablement présentant une cartouche de ciment osseux (132) pour le mélange de composants de départ de ciment osseux en polyméthacrylate de méthyle et pour l'évacuation de pâte de ciment osseux en polyméthacrylate de méthyle mélangée hors de la cartouche de ciment osseux (132), de manière particulièrement préférée l'au moins une cartouche de ciment osseux (132) présentant l'au moins un réservoir de ciment et contenant l'au moins un réservoir de liquide monomère, l'au moins un réservoir de ciment et l'au moins un réservoir de liquide monomère étant disposés dans des zones séparées l'une de l'autre.

11. Ensemble de fabrication de différents espaceurs (38, 88, 188, 238, 288) par durcissement de pâte de ciment osseux (36, 86), les espaceurs (38, 88, 188, 238, 288) étant prévus pour remplacer temporairement, dans le domaine médical, une articulation ou une partie d'une articulation comprenant une surface articulée d'une tête de l'articulation, en particulier pour remplacer temporairement une articulation de la hanche ou une articulation de l'épaule, l'ensemble présentant au moins un dispositif selon l'une des revendications précédentes, l'ensemble présentant au moins deux moules de tête (5, 55, 105, 155, 205, 255) comportant des diamètres différents des surfaces intérieures sphériques (7, 57, 107, 157, 207, 257) et/ou présentant au moins deux moules de tige (1, 51, 151, 251) comportant des cavités (6, 56, 106, 156, 206, 256) de longueurs différentes dans la direction distale ou des diamètres intérieurs différents des cavités (6, 56, 106, 156, 206, 256).

12. Ensemble selon la revendication 11, **caractérisé en ce que** l'ensemble présente au moins deux noyaux métalliques (10, 60, 160, 260) de longueurs différentes de la partie de tête (14, 64, 164, 264) et/ou de la partie de tige (12, 62, 162, 262).

13. Procédé de fabrication d'un espaceur (38, 88, 188, 238, 288) pour le remplacement temporaire d'une articulation ou d'une partie d'une articulation, en particulier d'une articulation de la hanche ou d'une articulation de l'épaule, comprenant une surface articulée de l'articulation, le procédé étant réalisé avec un dispositif selon l'une des revendications 1 à 10 ou avec un ensemble selon l'une des revendications 11 ou 12, le procédé présentant les étapes suivantes :
A) verser une pâte de ciment osseux (36, 86) dans l'espace intérieur (2, 52, 152, 252) du moule de tige (1, 51, 151, 251) et verser une pâte de ciment osseux (36, 86) dans la cavité (6, 56, 106, 156, 206, 256) du moule de tête (5, 55, 105, 155, 205, 255) ;
B) insérer la partie de tête (14, 64, 164, 264) du noyau métallique (10, 60, 160, 260) dans la cavité (6, 56, 106, 156, 206, 256) du moule de tête (5, 55, 105, 155, 205, 255) remplie de pâte de ciment osseux (36, 86) en déplaçant la pâte de ciment osseux (36, 86) contenue dans ladite cavité jusqu'à ce que la paroi distale (9, 59, 109, 159) du moule de tête (5, 55, 105, 155, 205, 255) repose contre la surface proximale (18, 68, 168) de la nervure (16, 66, 166, 266) du noyau métallique (10, 60, 160, 260), et insérer la partie de tige (12, 62, 162, 262) du noyau métallique (10, 60, 160, 260) dans l'espace intérieur (2, 52, 152, 252) du moule de tige (1, 51, 151, 251) rempli de pâte de ciment osseux (36, 86) en déplaçant la pâte de ciment osseux (36, 86) contenue dans ledit espace intérieur jusqu'à ce que la paroi proximale (4, 54, 154) du moule de tige (1, 51, 151, 251) repose contre la surface distale (20, 70, 170) de la nervure (16, 66, 166, 266) du noyau métallique (10, 60, 160, 260) ;
C) durcir la pâte de ciment osseux (36, 86) dans le moule de tête (5, 55, 105, 155, 205, 255) et le moule de tige (1, 51, 151, 251) ; et
D) retirer l'espaceur (38, 88, 188, 238, 288) ainsi formé et durci du moule de tête (5, 55, 105, 155, 205, 255) et du moule de tige (1, 51, 151, 251).

14. Procédé selon la revendication 13, **caractérisé en ce qu'**avant l'étape A), un moule de col (28, 78, 128, 178, 228, 278) du moule de tête (5, 55, 105, 155, 205, 255) correspondant à une longueur souhaitée du col (44, 94, 194, 244, 294) de l'espaceur (38, 88, 188, 238, 288) à fabriquer est raccourci, un moule de tête (5, 55, 105, 155, 205, 255) comportant un diamètre intérieur adapté de la surface intérieure sphérique (7, 57, 107, 157, 207, 257) est sélectionné pour être adapté à la forme souhaitée de la tête (40, 90, 190, 240, 290) de l'espaceur (38, 88, 188, 238, 288) à fabriquer, un moule de tige (1, 51, 151, 251) comportant un espace intérieur (2, 52, 152, 252) adapté à la forme souhaitée de la tige (42, 92, 192, 242, 292) de l'espaceur (38, 88, 188, 238, 288) à fabriquer est sélectionné et/ou un noyau métallique (10, 60, 160, 260) comportant des dimensions adaptées à la forme souhaitée de la tige (42, 92, 192, 242, 292) de l'espaceur (38, 88, 188, 238, 288) à fabriquer est sélectionné, et/ou avant l'étape A), la pâte de ciment osseux (36, 86) homogène est fabriquée en mélangeant un liquide monomère et une poudre de ciment, plus préférablement à l'étape A), la pâte de ciment osseux (36, 86) mélangée est pressée avec une cartouche de ciment osseux dans l'espace intérieur (2, 52, 152, 252) du moule de tige (1, 51, 151, 251) et dans la cavité (6, 56, 106, 156, 206, 256) du moule de tête (5, 55, 105, 155, 205, 255), de manière particulièrement préférée l'air est ainsi expulsé de l'espace intérieur (2, 52, 152, 252) du moule de tige (1, 51, 151, 251) et de la cavité (6, 56, 106, 156, 206, 256) du moule de tête (5, 55, 105, 155, 205, 255).

15. Procédé selon l'une des revendications 13 ou 14, **caractérisé en ce qu'**à l'étape B), la partie de tête (14, 64, 164, 264) du noyau métallique (10, 60, 160, 260) est centrée dans le moule de tête (5, 55, 105, 155, 205, 255) à l'aide d'appareils de centrage (24, 74,174, 274), en particulier à l'aide d'ailettes de centrage qui font saillie depuis la surface de la partie de tête (14, 64, 164, 264) et qui, lors de l'insertion de la partie de tête (14, 64, 164, 264) dans la cavité (6, 56, 106, 156, 206, 256) du moule de tête (5, 55, 105, 155, 205, 255), reposent contre des parois intérieures de la cavité (6, 56, 106, 156, 206, 256) du moule de tête (5, 55, 105, 155, 205, 255), et/ou à l'étape B), la partie de tige (12, 62, 162, 262) du noyau métallique (10, 60, 160, 260) est centrée dans le moule de tige (1, 51, 151, 251) à l'aide d'entretoises (22, 72, 172, 272) qui font saillie depuis la surface de la partie de tige (12, 62, 162, 262) et qui, lors de l'insertion de la partie de tige (12, 62, 162, 262) dans l'espace intérieur (2, 52, 152, 252) du moule de tige (1, 51, 151, 251), reposent contre des parois intérieures de l'espace intérieur (2, 52, 152, 252) du moule de tige (1, 51, 151, 251).
